# EUROPEAN PATENT APPLICATION

(11) **EP 4 670 616 A1**
(43) Date of publication of application: **31.12.2025**
(21) Application number: 23930837.2
(22) Date of filing: 17.11.2023
(51) Int. Cl.: A61B 3/15

(54) **OPHTHALMIC DEVICE AND OPHTHALMIC DEVICE OPERATING METHOD**

(30) Priority: 28.03.2023 JP 2023052055
(71) Applicant: Topcon Corporation, Tokyo 174-8580 (JP)
(72) Inventor: SHIBANO Takaaki, Tokyo 174-8580 (JP); MOCHIZUKI Yuki, Tokyo 174-8580 (JP); SUZUKI Minami, Tokyo 174-8580 (JP); YAMABE Masaru, Tokyo 174-8580 (JP); TSUKIHARA Kouichi, Tokyo 174-8580 (JP)
(74) Representative: Schmitt-Nilson Schraud Waibel Wohlfrom Patentanwälte Partnerschaft mbB
(86) International application number: PCT/JP2023/041450
(87) International publication number: WO 2024/202210

(57) **Abstract**

There are provided an ophthalmic device capable of examining on a subject eye by an examination head without bringing the examination head close to a nose of a subject and a method for operating the ophthalmic device. The ophthalmic device includes an examination head (22) configured to examine a subject eye (E) and a displacement mechanism (an XZ movement mechanism (16), a Y movement mechanism (18), a swing rotation mechanism (20), and a tilt rotation mechanism (80)) configured to displace the examination head (22) with respect to the subject eye (E). The displacement mechanism displaces the examination head (22) to an examination position for the subject eye (E) along an axis (TA) of tilt. Here, the axis (TA) of tilt is an axis obtained by tilting, around the center of the subject eye, a reference axis (VA) that extends along the eye direction of the subject eye (E) in parallel with a front-back direction serving as an operating distance direction (Z-direction) of the examination head (22), outward away from a subject's nose.

## Description

### {Technical Field}

The presently disclosed subject matter relates to an ophthalmic device which aligns an examination head with a subject eye and a method for operating the ophthalmic device.

### {Background Art}

In ophthalmology, ophthalmic examinations (e.g., acquisition of various ocular characteristics, such as ocular refractive power, an intraocular pressure, and the number of corneal endothelial cells, of a subject eye, fundus imaging, and tomography) on the subject eye are performed by an ophthalmic device. Alignment of an examination head (also referred to as an examination unit) of the ophthalmic device with respect to the subject eye is extremely important in terms of, e.g., accuracy, reliability, and image quality of a result of the examinations on the subject eye.

For this reason, each of the ophthalmic devices according to Patent Literatures 1 and 2 stereoscopically photographs a subject eye using a stereo camera and executes alignment detection that detects a relative position of the subject eye to an examination head based on anterior eye part images of the subject eye obtained by the stereoscopic photographing. The ophthalmic device moves the examination head through electromotive driving based on a result of the alignment detection, thereby executing automatic alignment of the examination head with the subject eye.

### {Citation List}

### {Patent Literature}

{Patent Literature 1} Japanese Patent Application Laid-Open No. 2013-248376
{Patent Literature 2} Japanese Patent Application Laid-Open No. 2021-069415

### {Summary of Invention}

### {Technical Problem}

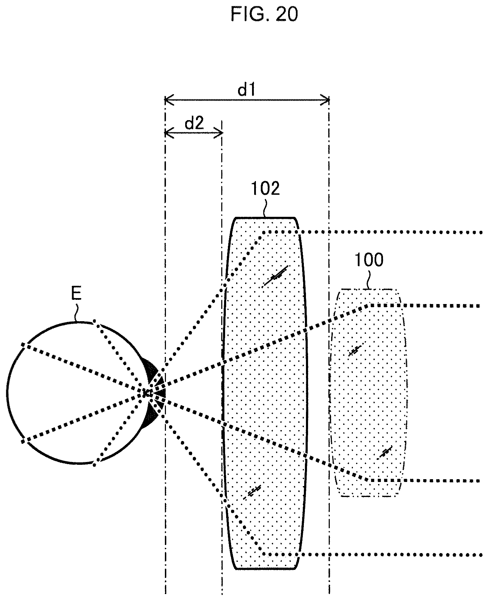

Figure 20 is an explanatory diagram for explaining a relationship between a lens size of an objective lens in an examination head and an operating distance of the examination head. As illustrated in Figure 20, a photographic angle of view of a conventional objective lens 100 in an examination head is about 45°. An ophthalmic device such as a fundus camera, an Optical Coherence Tomography (OCT) device, or a Scanning Laser Ophthalmoscope (SLO) device supporting wide-angle photographing, has an objective lens 102 larger than the objective lens 100 in an examination head due to optical constraints. As a result, an operating distance d2 between a subject eye E and the examination head with the objective lens 102 is shorter than an operating distance d1 with the objective lens 100.

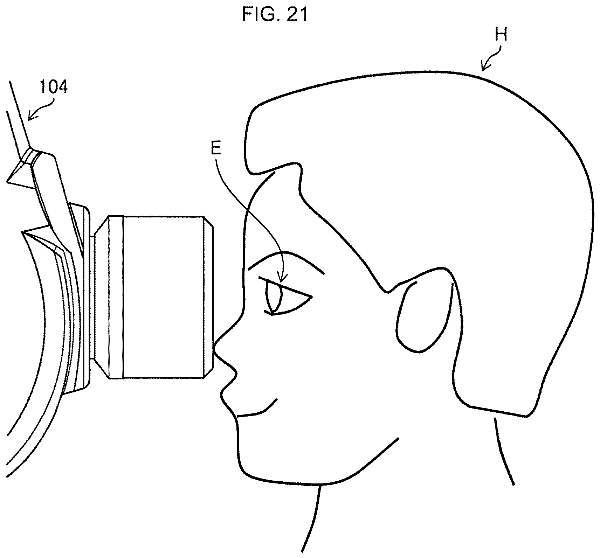

Figure 21 is an explanatory view for explaining a problem arising from a short operating distance between the subject eye E and an examination head 104. For example, in a case where an objective lens diameter of the examination head 104 is 80 mm, and an operating distance of the examination head 104 is 50 mm, as illustrated in Figure 21, a distance between a forehead of the subject H and the examination head 104 is about 22 mm, a distance between a nose of the subject H and the examination head 104 is about 2 mm, and a distance between cheeks of the subject H and the examination head 104 is about 28 mm. The distance between the nose of the subject H and the examination head 104 is thus particularly short. As a result, in executing automatic alignment of the examination head 104 with the subject eye E using the methods according to Patent Literatures 1 and 2 described above, the examination head 104 needs to be brought close to the nose of the subject H.

The presently disclosed subject matter has been made in view of the above-described circumstances, and has as its object to provide an ophthalmic device capable of examining on a subject eye through an examination head without bringing the examination head close to a nose of a subject and a method for operating the ophthalmic device.

### {Solution to Problem}

An ophthalmic device for achieving the object of the presently disclosed subject matter includes: an examination head configured to examine a subject eye; and a displacement mechanism configured to displace the examination head with respect to the subject eye. The displacement mechanism is configured to displace the examination head to an examination position for the subject eye along an axis of tilt. Here, the axis of tilt is an axis obtained by tilting, around the center of the subject eye, a reference axis that extends along the eye direction of the subject eye in parallel with a front-back direction serving as an operating distance direction of the examination head, outward away from a subject's nose.

According to the ophthalmic device, it is possible to displace the examination head to the examination position for the subject eye without bringing the examination head close to the subject's nose.

In the ophthalmic device according to another aspect of the presently disclosed subject matter, the displacement mechanism displaces the examination head to the examination position while keeping a tilting angle of the axis of tilt with respect to the reference axis constant. This makes it possible to displace the examination head to the examination position for the subject eye without bringing the examination head close to the subject's nose.

In the ophthalmic device according to another aspect of the presently disclosed subject matter, the displacement mechanism includes a movement mechanism configured to move the examination head in the front-back direction, a left-right direction, and an up-down direction with respect to the subject eye, and a rotation mechanism configured to rotate the examination head around a rotation axis determined in advance. This makes it possible to arbitrarily displace the examination head with respect to the subject eye.

In the ophthalmic device according to another aspect of the presently disclosed subject matter, the rotation axis is parallel to the up-down direction, and the outward direction is parallel to the left-right direction.

In the ophthalmic device according to another aspect of the presently disclosed subject matter, the rotation axis is perpendicular to the up-down direction, and the outward direction is an upward direction of the up-down direction.

In the ophthalmic device according to another aspect of the presently disclosed subject matter, the examination head includes an objective lens, the rotation mechanism rotates the examination head around the objective lens, and the movement mechanism integrally moves the examination head and the rotation axis in the front-back direction, the left-right direction, and the up-down direction.

In the ophthalmic device according to another aspect of the presently disclosed subject matter, the rotation axis is parallel to the up-down direction, and the outward direction is parallel to the left-right direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of driving the movement mechanism to move the examination head from the initial position to the axis of tilt in the outward direction, a second driving process of driving the rotation mechanism to rotate the examination head around the rotation axis and make the optical axis of the objective lens parallel to the axis of tilt, and a third driving process of driving the movement mechanism after completion of the first driving process and the second driving process to move the examination head to the examination position along the axis of tilt. This makes it possible to displace the examination head to the examination position for the subject eye without bringing the examination head close to the subject's nose.

In the ophthalmic device according to another aspect of the presently disclosed subject matter, the rotation axis is parallel to the up-down direction, and the outward direction is parallel to the left-right direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of driving the movement mechanism to move the examination head from the initial position toward a front side in the front-back direction which faces the subject eye and then move the examination head to the axis of tilt in the outward direction, a second driving process of driving the rotation mechanism to rotate the examination head around the rotation axis and make the optical axis of the objective lens parallel to the axis of tilt, and a third driving process of driving the movement mechanism after completion of the first driving process and the second driving process to move the examination head to the examination position along the axis of tilt. This makes it possible to displace the examination head to the examination position for the subject eye without bringing the examination head close to the subject's nose, and to reduce an amount of movement of the examination head in the outward direction in the first driving process.

In the ophthalmic device according to another aspect of the presently disclosed subject matter, the rotation axis is parallel to the up-down direction, and the outward direction is parallel to the left-right direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of driving the movement mechanism and the rotation mechanism to simultaneously execute movement of the examination head toward a front side in the front-back direction which faces the subject eye, movement of the examination head in the outward direction, and rotation of the examination head around the rotation axis, and move the examination head to the axis of tilt and make the optical axis of the objective lens parallel to the axis of tilt, and a second driving process of driving the movement mechanism after completion of the first driving process to move the examination head to the examination position along the axis of tilt. This makes it possible to displace the examination head to the examination position for the subject eye without bringing the examination head close to the subject's nose, and to displace the examination head to the examination position in a shortest time.

In the ophthalmic device according to another aspect of the presently disclosed subject matter, the rotation axis is perpendicular to the up-down direction, and the outward direction is an upward direction of the up-down direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of driving the movement mechanism and the rotation mechanism to move the examination head to the axis of tilt and make the optical axis of the objective lens parallel to the axis of tilt, and a second driving process of driving the movement mechanism after completion of the first driving process to move the examination head to the examination position along the axis of tilt. This makes it possible to displace the examination head to the examination position for the subject eye without bringing the examination head close to the subject's nose.

In the ophthalmic device according to another aspect of the presently disclosed subject matter, the examination head includes an objective lens, the rotation axis is located on a front side in the front-back direction which is closer to the subject eye than the objective lens is, and the movement mechanism integrally moves the examination head and the rotation axis in the front-back direction, the left-right direction, and the up-down direction.

In the ophthalmic device according to another aspect of the presently disclosed subject matter, the rotation axis is parallel to the up-down direction, and the outward direction is parallel to the left-right direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of driving the movement mechanism to move the examination head to a position where the rotation axis coincides with the subject eye when the examination head is viewed from the one-direction side, a second driving process of driving the rotation mechanism after completion of the first driving process to move the examination head to the axis of tilt and make the optical axis of the objective lens parallel to the axis of tilt by rotating the examination head around the rotation axis, and a third driving process of driving the movement mechanism after completion of the second driving process to move the examination head to the examination position along the axis of tilt. This makes it possible to displace the examination head to the examination position for the subject eye without bringing the examination head close to the subject's nose.

In the ophthalmic device according to another aspect of the presently disclosed subject matter, the rotation axis is parallel to the up-down direction, and the outward direction is parallel to the left-right direction, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of simultaneously executing a process of driving the movement mechanism to move the examination head to a position where the rotation axis coincides with the subject eye when the examination head is viewed from the one-direction side and a process of driving the rotation mechanism to rotate the examination head around the rotation axis, and moving the examination head to the axis of tilt and making the optical axis of the objective lens parallel to the axis of tilt, and a second driving process of driving the movement mechanism after completion of the first driving process to move the examination head to the examination position along the axis of tilt. This makes it possible to displace the examination head to the examination position for the subject eye without bringing the examination head close to the subject's nose, and to displace the examination head to the examination position in a shortest time.

In the ophthalmic device according to another aspect of the presently disclosed subject matter, the rotation mechanism is capable of rotating the examination head around a first rotation axis and a second rotation axis constituting the rotation axis, the first rotation axis being parallel to the up-down direction, the second rotation axis being perpendicular to the up-down direction.

In the ophthalmic device according to another aspect of the presently disclosed subject matter, an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction, the ophthalmic device includes a drive controlling unit configured to control driving of the displacement mechanism, and the drive controlling unit executes a first driving process of executing a process of driving the movement mechanism to move the examination head to a position where the first rotation axis and the second rotation axis coincide with the subject eye and a process of driving the rotation mechanism to rotate the examination head around at least one of the first rotation axis and the second rotation axis, and moving the examination head to the axis of tilt and making the optical axis of the objective lens parallel to the axis of tilt, and a second driving process of driving the movement mechanism after completion of the first driving process to move the examination head to the examination position along the axis of tilt. This makes it possible to displace the examination head to the examination position for the subject eye without bringing the examination head close to the subject's nose.

The ophthalmic device according to another aspect of the presently disclosed subject matter includes a vision fixation light emitting unit configured to emit vision fixation light. This makes it possible to cause the eye direction of the subject eye to follow displacement of the examination head.

The ophthalmic device according to another aspect of the presently disclosed subject matter includes a photographing unit provided at the examination head and configured to photograph an anterior eye part of the subject eye halfway through displacement of the examination head by the displacement mechanism, an alignment detection unit configured to detect a relative position of the subject eye to the examination head based on an anterior eye part image of the subject eye photographed by the photographing unit, and a drive controlling unit configured to control driving of the displacement mechanism, and the drive controlling unit drives the displacement mechanism based on a detection result from the alignment detection unit to move the examination head to the examination position along the axis of tilt. This makes it possible to displace the examination head to the examination position for the subject eye without bringing the examination head close to the subject's nose.

The ophthalmic device according to another aspect of the presently disclosed subject matter includes a manipulation unit, and the drive controlling unit is switchable to a manual control mode of driving the displacement mechanism in accordance with an input manipulation to the manipulation unit to displace the examination head, and the drive controlling unit switches to the manual control mode in a case where detection of the relative position by the alignment detection unit is not executed while the examination head is moved for a fixed time determined in advance or over a fixed distance by the displacement mechanism. This makes it possible to automatically switch to the manual control mode in a case where the alignment detection unit is incapable of detection of the relative position due to, for example, individual difference in face, a diseased eye, a drooping eyelid, or the effect of eyelashes.

In the ophthalmic device according to another aspect of the presently disclosed subject matter, the photographing unit includes a plurality of cameras capable of photographing the subject eye from a plurality of directions different from each other.

A method for operating an ophthalmic device for achieving the object of the presently disclosed subject matter is a method for operating an ophthalmic device, the ophthalmic device including an examination head configured to examine a subject eye, and a displacement mechanism including a rotation mechanism configured to displace the examination head with respect to the subject eye. The method includes driving the displacement mechanism to displace the examination head to an examination position for the subject eye along an axis of tilt. Here, the axis of tilt is an axis obtained by tilting, around the center of the subject eye, a reference axis that extends along the eye direction of the subject eye in parallel with a front-back direction serving as an operating distance direction of the examination head, outward away from a subject's nose.

### {Advantageous Effects of Invention}

According to the presently disclosed subject matter, it is possible to examine a subject eye by an examination head without bringing the examination head close to a subject's nose.

### {Brief Description of Drawings}

Figure 1 is a side view of an ophthalmic device according to a first embodiment;
Figure 2 is a front view of a lens-barrel as viewed from a front side in a Z direction;
Figure 3 is a cross-sectional view of the lens-barrel taken along line 3-3 in Figure 2;
Figure 4 is a block diagram illustrating a configuration of the ophthalmic device according to the first embodiment;
Figure 5 is an explanatory diagram for explaining a method for automatic alignment of an examination head;
Figure 6 is an explanatory diagram for explaining an example 1-1 of the automatic alignment of the examination head according to the first embodiment;
Figure 7 is an explanatory diagram for explaining an example 1-2 of the automatic alignment of the examination head according to the first embodiment;
Figure 8 is an explanatory diagram for explaining an example 1-3 of the automatic alignment of the examination head according to the first embodiment;
Figure 9 is a flowchart illustrating a flow of a process of examining a subject eye by the ophthalmic device according to the first embodiment;
Figure 10 is a flowchart illustrating a flow of an automatic alignment process for the examination head;
Figure 11 is an explanatory diagram for explaining displacement of the examination head after the start of the automatic alignment;
Figure 12 is a flowchart illustrating a flow of a modification of the automatic alignment of the examination head;
Figure 13 is a side view of an ophthalmic device according to a second embodiment;
Figure 14 is an explanatory diagram for explaining an example 2-1 of automatic alignment of an examination head according to the second embodiment;
Figure 15 is an explanatory diagram for explaining an example 2-2 of the automatic alignment of the examination head according to the second embodiment;
Figure 16 is a side view of an ophthalmic device according to a third embodiment;
Figure 17 is an explanatory diagram for explaining an example 3 of automatic alignment of an examination head according to the third embodiment;
Figure 18 is a side view of an ophthalmic device according to a fourth embodiment;
Figure 19 is an explanatory diagram for explaining an example 4 of automatic alignment of an examination head according to the fourth embodiment;
Figure 20 is an explanatory diagram for explaining a relationship between a lens size of an objective lens in an examination head and an operating distance of the examination head; and
Figure 21 is an explanatory view for explaining a problem arising from a short operating distance between a subject eye and an examination head.

### {Description of Embodiments}

### [First Embodiment]

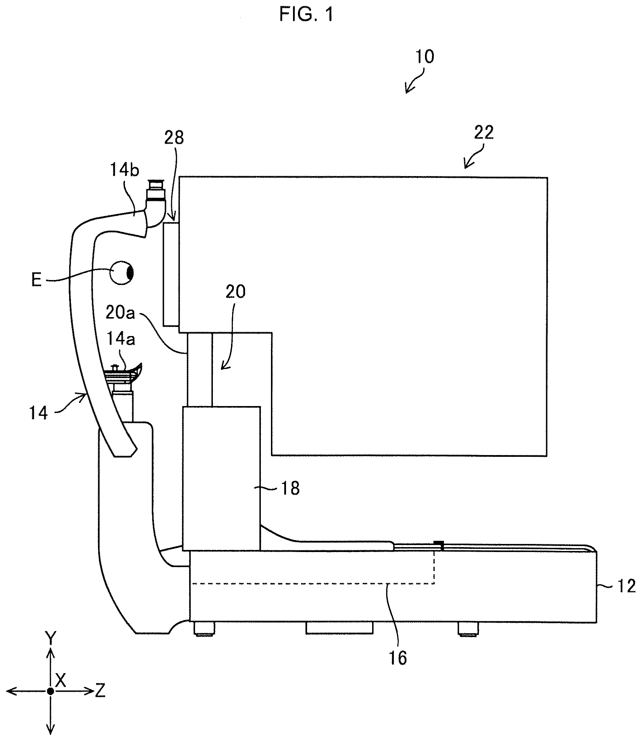

Figure 1 is a side view of an ophthalmic device 10 according to a first embodiment. An X direction in Figure 1 is a left-right direction based on a subject, a Y direction is an up-down direction, and that a Z direction is a front-back direction (also referred to as an operating distance direction) parallel to a forward direction toward the subject (a subject eye E) and a rearward direction away from the subject.

As illustrated in Figure 1, the ophthalmic device 10 is a multifunction machine which is a combination of a fundus camera that executes fundus imaging of the subject eye E and an optical coherence tomograph that obtains a tomographic image of the subject eye E using OCT. The ophthalmic device 10 includes a base 12, a face support 14, an XZ movement mechanism 16, a Y movement mechanism 18, a swing rotation mechanism 20, and an examination head 22.

The face support 14 is attached to a front end portion on a front side (a subject eye E side) in the Z direction of the base 12. The XZ movement mechanism 16 is also provided at the base 12.

The face support 14 includes a chin rest 14a and a forehead rest 14b which are positionally adjustable in the Y direction (up-down direction), and supports a face of the subject at a position facing the examination head 22 (a lens-barrel 28).

The XZ movement mechanism 16 together with the Y movement mechanism 18 (to be described later) constitutes a movement mechanism according to the presently disclosed subject matter. The XZ movement mechanism 16 includes a pedestal which is movable in each of the X and Z directions with respect to the base 12 and an electric drive mechanism (a publicly known actuator, such as a motor drive mechanism) which moves the pedestal in each of the X and Z directions (both not illustrated). The XZ movement mechanism 16 integrally moves the Y movement mechanism 18, the swing rotation mechanism 20, and the examination head 22 in the X and Z directions.

The Y movement mechanism 18 includes a lifting table which is be movable in the Y direction and an electric drive mechanism which moves the lifting table in the Y direction (both not illustrated). The Y movement mechanism 18 integrally moves the swing rotation mechanism 20 and the examination head 22 in the Y direction. With this configuration, the XZ movement mechanism 16 and the Y movement mechanism 18 can integrally move the swing rotation mechanism 20 and the examination head 22 in the X, Y, and Z directions.

The swing rotation mechanism 20 corresponds to a rotation mechanism according to the presently disclosed subject matter and, together with the XZ movement mechanism 16 and the Y movement mechanism 18 described earlier, constitutes a displacement mechanism according to the presently disclosed subject matter. The swing rotation mechanism 20 includes a rotating shaft 20a parallel to the Y direction and an electric drive mechanism which rotates the rotating shaft 20a, and rotates (swings) the examination head 22 around the rotating shaft 20a.

The examination head 22 is attached to an upper end portion in the Y direction of the rotating shaft 20a. With this configuration, the examination head 22 is movable in the X, Y, and Z direction by the XZ movement mechanism 16 and the Y movement mechanism 18, and is rotatable in a direction around the rotating shaft 20a by the swing rotation mechanism 20.

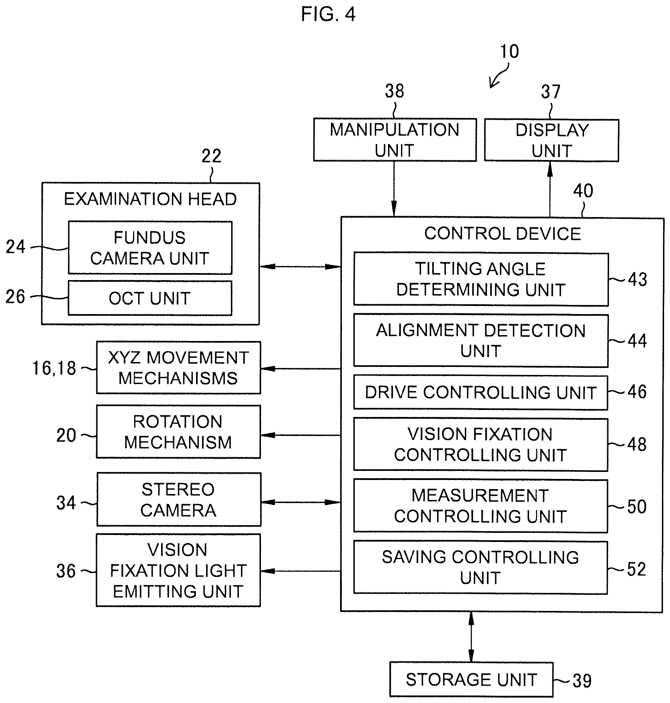

The examination head 22 includes a fundus camera unit 24 and an OCT unit 26 (to be described later) illustrated in Figure 4, and the lens-barrel 28. The fundus camera unit 24 photographs a fundus of the subject eye E through an objective lens 30 (to be described later) and outputs a fundus image which is a frontal image of the fundus to a control device 40 (to be described later) (see Figure 4). The OCT unit 26 performs OCT imaging of the subject eye E through the objective lens 30 and outputs a detection signal needed to generate a tomographic image of the subject eye E to the control device 40. Specific configurations of the fundus camera unit 24 and the OCT unit 26 are publicly known techniques (see Patent Literature 1 described above) and that a specific description thereof will be omitted.

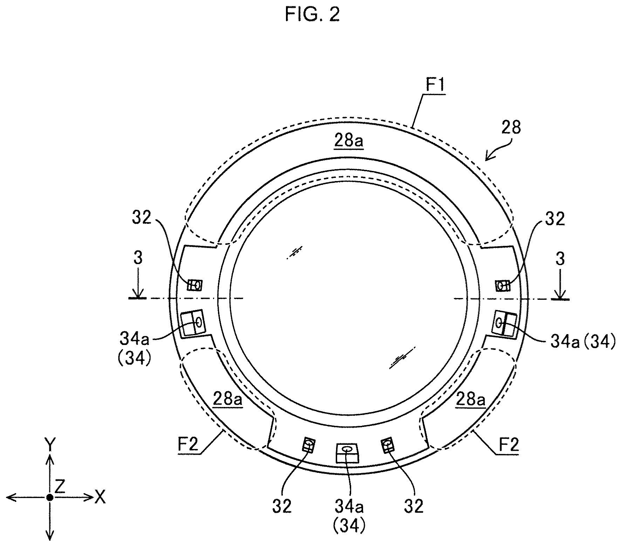

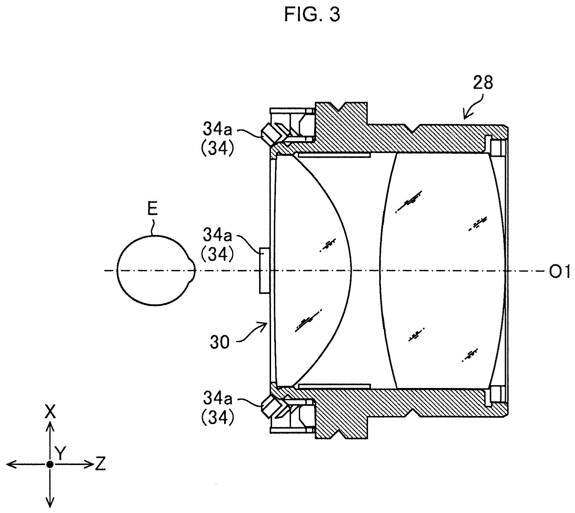

Figure 2 is a front view of the lens-barrel 28 as viewed from the front side in the Z direction. Figure 3 is a cross-sectional view of the lens-barrel 28 taken along line 3-3 in Figure 2. Figures 1 to 3 illustrate an example which provides the lens-barrel 28 at an end portion on the front side in the Z direction of the examination head 22. The lens-barrel 28 houses (holds) the objective lens 30 having an optical axis O1 (see Figure 3) parallel to the Z direction. Four illumination light sources 32 and a stereo camera 34 are provided at a lens-barrel distal end face 28a on the front side in the Z direction of the lens-barrel 28. As the objective lens 30, for example, a large lens supporting wide-angle photographing, i.e., a lens with a short operating distance is used (see Figure 20). The type of the objective lens 30 is not particularly limited and that a lens with a photographic angle of view of about 45° may be used.

When the objective lens 30 and the rotating shaft 20a described earlier are viewed from a one-direction side in the Y direction, a position of the rotating shaft 20a and a position of the objective lens 30 coincide (the term "coincide" as used herein is intended to include the meaning of "substantially coincide"; the same applies hereinafter). With this configuration, the examination head 22 is rotated (swung) around the objective lens 30 by the swing rotation mechanism 20.

Respective illumination light sources 32 are provided at two end portions in the X direction (two left and right end portions) of the lens-barrel distal end face 28a, and two illumination light sources 32 are provided at a lower end portion of the lens-barrel distal end face 28a such that a camera 34a (to be described later) is sandwiched therebetween. Each illumination light source 32 is, for example, an LED (Light Emitting Diode) light source and illuminates the subject eye E.

The stereo camera 34 corresponds to a photographing unit according to the presently disclosed subject matter and is used for alignment detection that detects relative positions in the X, Y, and Z directions of the subject eye E to the examination head 22. The stereo camera 34 includes a plurality of cameras 34a. For example, in the present embodiment, the stereo camera 34 includes two cameras 34a provided at the two end portions in the X direction (the two left and right end portions) corresponding to positions on the left and right of the objective lens 30 in the lens-barrel distal end face 28a and one camera 34a provided at the lower end portion corresponding to a position below the objective lens 30 in the lens-barrel distal end face 28a, three cameras 34a in total. The cameras 34a simultaneously photograph an anterior eye part of the subject eye E from a plurality of (three in the present embodiment) directions different from each other at the time of the alignment detection and output a plurality of (three) anterior eye part images of the subject eye E to the control device 40 (see Figure 4). The number of cameras 34a may be two, or four or more.

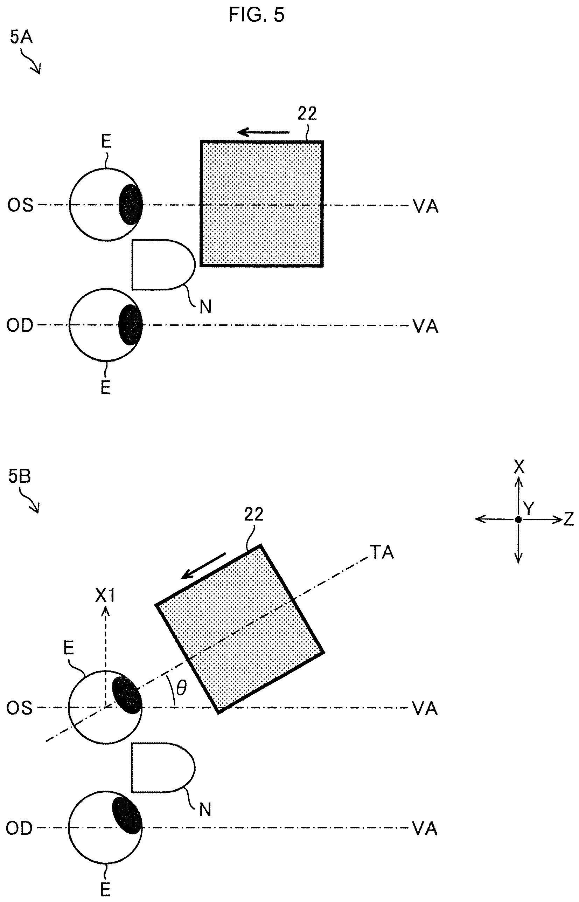

Positions of the cameras 34a may be appropriately changed. For example, in a case where the camera 34a is provided in an upper region F1 (see Figure 2) above (in the Y direction) the two end portions in the X direction in the lens-barrel distal end face 28a, a pupil of the subject eye E may be hidden by upper lashes of the subject in photographing the subject eye E by the camera 34a. Additionally, in a case where respective cameras 34a are provided in left and right lower oblique regions F2 (see Figure 2) between the two end portions in the X direction and the lower end portion in the lens-barrel distal end face 28a, each camera 34a is likely to come close to a subject's nose N (see Figure 5) during alignment of the examination head 22. For this reason, the cameras 34a are preferably provided at the two end portions in the X direction and the lower end portion of the lens-barrel distal end face 28a.

Figure 4 is a block diagram illustrating a configuration of the ophthalmic device 10 according to the first embodiment. As illustrated in Figure 4, the ophthalmic device 10 includes a vision fixation light emitting unit 36, a display unit 37, a manipulation unit 38, a storage unit 39, and the control device 40 in addition to the XZ movement mechanism 16, the Y movement mechanism 18, the swing rotation mechanism 20, the examination head 22, and the stereo camera 34 described earlier.

The vision fixation light emitting unit 36 guides and fixes an eye direction of the subject eye E by emitting vision fixation light (a bright spot image) toward the subject eye E. The vision fixation light emitting unit 36 includes a publicly known vision fixation target display unit, a plurality of vision fixation holes, and an external fixation lamp (all not illustrated) (see Patent Literature 2 described above).

The vision fixation target display unit is provided inside the examination head 22 and is used for internal vision fixation that projects vision fixation light (e.g., a bright spot image) onto the subject eye E through the objective lens 30. The vision fixation holes are provided at a front surface in the Z direction (which may be the lens-barrel distal end face 28a) of the examination head 22 so as to surround the objective lens 30 and are used for peripheral vision fixation. The peripheral vision fixation is a vision fixation method for selectively lighting the vision fixation holes, thereby causing the subject eye E to make a great circumnutation in a desired direction. The external fixation lamp is provided at the face support 14 or the examination head 22 and is used for external vision fixation. The external vision fixation is a vision fixation method for causing the subject eye E to make a circumnutation in an arbitrary direction or make a greater circumnutation than under internal vision fixation by adjusting a light source position of the external fixation lamp, or adjusting an orientation of the subject eye E by guiding a visual line of the subject eye E or a fellow eye when the internal vision fixation cannot be performed.

As the display unit 37, which is a type of display device, for example, a touch-panel monitor is used. The display unit 37 displays screens such as a setup screen for the ophthalmic device 10, a manipulation screen (User Interface (UI) screen) for the ophthalmic device 10, anterior eye part images of the subject eye E photographed by the stereo camera 34, a result (a fundus image and a tomographic image of the subject eye E) of examining the subject eye E by the examination head 22.

Examples of the manipulation unit 38 includes a publicly known manipulation lever, switches and a manipulation screen to be displayed on the display unit 37 (all not illustrated). The manipulation unit 38 is used to input an instruction such as a positional adjustment of the chin rest 14a and the forehead rest 14b, an XYZ movement and a rotation of the examination head 22, selecting the type of examination (from fundus imaging and OCT imaging), switching between automatic alignment and manual alignment, an examination start, or saving an examination result (a fundus image or a tomographic image).

The storage unit 39 is a recording medium (storage medium) which stores a program to be executed by the control device 40, and various publicly known storages are used as the storage unit 39. A fundus image of the subject eye E photographed by the fundus camera unit 24 and a tomographic image of the subject eye E obtained through OCT imaging by the OCT unit 26 are saved in the storage unit 39.

The control device 40 performs overall control on the action of the units of the ophthalmic device 10 and executes the control such as alignment of the examination head 22 with the subject eye E, imaging of the fundus of the subject eye E by the fundus camera unit 24, and OCT imaging of the subject eye E by the OCT unit 26.

The control device 40 includes an arithmetic circuit including various processors and memories. Examples of the various processors include a Central Processing Unit (CPU), a Graphics Processing Unit (GPU), an Application Specific Integrated Circuit (ASIC), programmable logic devices (e.g., a Simple Programmable Logic Devices (SPLD), a Complex Programmable Logic Device (CPLD), and a Field Programmable Gate Arrays (FPGA)). Various functions of the control device 40 may be implemented by one processor or may be implemented by a plurality of processors of the same type or of different types.

The control device 40 functions as a tilting angle determining unit 43, an alignment detection unit 44, a drive controlling unit 46, a vision fixation controlling unit 48, a measurement controlling unit 50, and a saving controlling unit 52 by executing a control program stored in the storage unit 39.

Figure 5 is an explanatory diagram for explaining a method for automatic alignment of the examination head 22. Here, reference character "OD" in Figure 5 denotes a right eye (oculus dexter) and that reference character "OS" denotes a left eye (oculus sinister). As described earlier, in the ophthalmic device 10 (the fundus camera and the OCT device) supporting wide-angle photographing, an operating distance of the examination head 22 is short due to the objective lens 30 of large size. For this reason, as indicated by reference character 5A in Figure 5, when the examination head 22 is moved from a position in front of the subject eye E (the left eye OS here) toward the front side in the Z direction at the time of the automatic alignment of the examination head 22, the examination head 22 may come close to the subject's nose N.

For the above-described reason, as indicated by reference character 5B in Figure 5, when viewed from the one-direction side in the Y direction, the ophthalmic device 10 according to the present embodiment brings the examination head 22 is brought closer to the subject eye E from an oblique direction during the automatic alignment of the examination head 22.

Specifically, assume that an axis along the eye direction (an eye direction of the subject eye E observing infinite distance) of the subject eye E parallel to the Z direction is a reference axis VA, that for X direction, the outward direction away from the nose (N), based on the subject eye E (here, the left eye OS), is defined as X1. Furthermore, an axis obtained by tilting the reference axis VA in the outward direction X1 around the subject eye E is designated as an axis TA of tilt. The examination head 22 is displaced to an examination position where examination (fundus imaging and OCT imaging) of the subject eye E is executable (hereinafter simply referred to as the examination position) along the axis TA of tilt when viewed from the one-direction side in the Y direction. The term "displacement" here subsumes movement in the X, Y, and Z directions and rotation of the examination head 22.

The tilting angle determining unit 43 determines a tilting angle θ of the axis TA of tilt with respect to the reference axis VA when viewed from the one-direction side in the Y direction, i.e., the tilting angle θ of the axis TA of tilt with respect to the reference axis VA in an XZ plane. For example, the tilting angle determining unit 43 determines, as the tilting angle θ, a value that an examiner selects from a plurality of angles (e.g., 10°, 15°, and 20°) with the manipulation unit 38 and outputs information on the tilting angle θ to the drive controlling unit 46. The tilting angle determining unit 43 may detect a relative position of the nose N to the examination head 22 based on photographed images obtained through stereoscopic photographing of the nose N by the stereo camera 34 and determine the tilting angle θ that can prevent the examination head 22 from coming close to the nose N based on a result of the detection.

Referring back to Figure 4, the alignment detection unit 44 detect a relative position of the subject eye E to the examination head 22 by identification of a pupil center position of the subject eye E and computation of three-dimensional coordinates of the pupil center position based on anterior eye part images of the subject eye E stereoscopically photographed by the cameras 34a of the stereo camera 34 during the automatic alignment of the examination head 22. Since a method for alignment detection using the stereo camera 34 is a publicly known technique (see Patent Literature 1 described above), a specific description thereof will be omitted.

The drive controlling unit 46 drives the XZ movement mechanism 16, the Y movement mechanism 18, and the swing rotation mechanism 20 to execute alignment of the examination head 22 with the subject eye E and switching of the subject eye E as an examination object (left-right eye switching). The alignment of the examination head 22 includes automatic alignment that is performed by automatically driving the XZ movement mechanism 16, the Y movement mechanism 18, and the swing rotation mechanism 20 and manual alignment (corresponding to a manual control mode according to the presently disclosed subject matter) that drives the XZ movement mechanism 16, the Y movement mechanism 18, and the swing rotation mechanism 20 in accordance with a manipulation input to the manipulation unit 38. The manipulation unit 38 is configured to execute switching between the automatic alignment and the manual alignment is executed with the manipulation unit 38.

The drive controlling unit 46 determines the axis TA of tilt corresponding to the tilting angle θ based on the tilting angle θ initially determined by the tilting angle determining unit 43 at the time of the automatic alignment.

For example, the drive controlling unit 46 identifies the reference axis VA based on photographed images obtained through initial stereoscopic photographing of the face (e.g., the subject eye E or the nose N) of the subject by the stereo camera 34. Alternatively, the drive controlling unit 46 estimates the reference axis VA based on positions in the Y direction of the chin rest 14a and the forehead rest 14b and identification information on the left eye OS and the right eye OD that are already known. The drive controlling unit 46 determines, as the axis TA of tilt, an axis obtained by tilting the reference axis VA in the outward direction X1 around the subject eye E by the tilting angle θ.

The drive controlling unit 46 then drives the XZ movement mechanism 16, the Y movement mechanism 18, and the swing rotation mechanism 20 based on the axis TA of tilt to start automatic alignment that automatically displaces the examination head 22 from an initial position when the ophthalmic device 10 is powered on to the examination position.

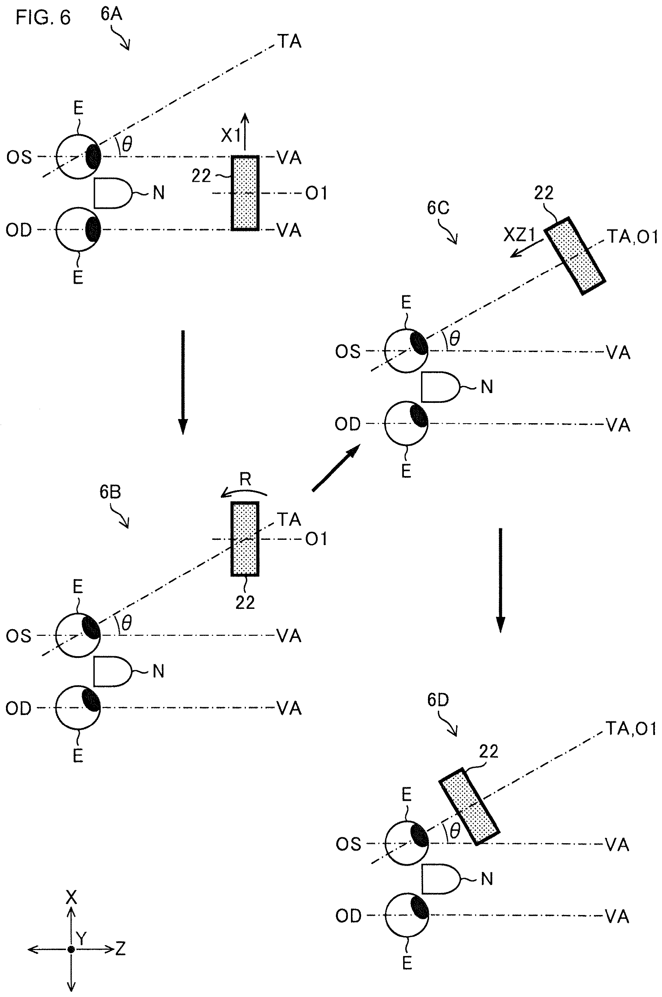

Figure 6 is an explanatory diagram for explaining an example 1-1 of the automatic alignment of the examination head 22 according to the first embodiment. As indicated by reference character 6A in Figure 6, the examination head 22 is initially arranged at a position where the optical axis O1 of the objective lens 30 is between the reference axes VA of the left and right subject eyes E (the left eye OS and the right eye OD) when viewed from the one-direction side in the Y direction, i.e., a position facing (the term "face" as used herein is intended to include the meaning of "substantially face"; the same applies hereinafter) the nose N.

First, the drive controlling unit 46 drives the XZ movement mechanism 16 to execute a first driving process of moving the examination head 22 from the initial position to the axis TA of tilt in the outward direction X1 when the examination head 22 is viewed from the one-direction side in the Y direction.

As indicated by reference character 6B in Figure 6, the drive controlling unit 46 drives the swing rotation mechanism 20 after completion of the first driving process to execute a second driving process of rotating the examination head 22 around the rotating shaft 20a by the tilting angle θ (see an arrow R). With this process, as indicated by reference character 6C in Figure 6, the optical axis O1 of the examination head 22 (the objective lens 30) becomes parallel to the axis TA of tilt. The second driving process may be executed before the first driving process.

The drive controlling unit 46 then drives the XZ movement mechanism 16 after completion of the second driving process to start a third driving process of moving the examination head 22 to the examination position along the axis TA of tilt when the examination head 22 is viewed from the one-direction side in the Y direction (see an arrow XZ1). With this process, the examination head 22 is moved toward the subject eye E while keeping the tilting angle θ constant (the term "constant" as used herein is intended to include the meaning of "substantially constant"; the same applies hereinafter).

Halfway through the automatic alignment, the examination head 22 is displaced to a position where the alignment detection unit 44 can identify the pupil center position of the subject eye E, i.e., a position where the alignment detection is possible. For this reason, an alignment detection result is input from the alignment detection unit 44 to the drive controlling unit 46. Y-direction positional adjustment of the examination head 22 by the Y movement mechanism 18 may be executed before the alignment detection (at any stage from the first driving process to the third driving process) such that the alignment detection by the alignment detection unit 44 is possible.

As indicated by reference character 6D in Figure 6, the drive controlling unit 46 drives the XZ movement mechanism 16 and the Y movement mechanism 18 based on an alignment detection result input from the alignment detection unit 44 to continue the third driving process until the examination head 22 reaches the examination position. In the third driving process that is executed based on the alignment detection result, the Y-direction positional adjustment of the examination head 22 is also executed.

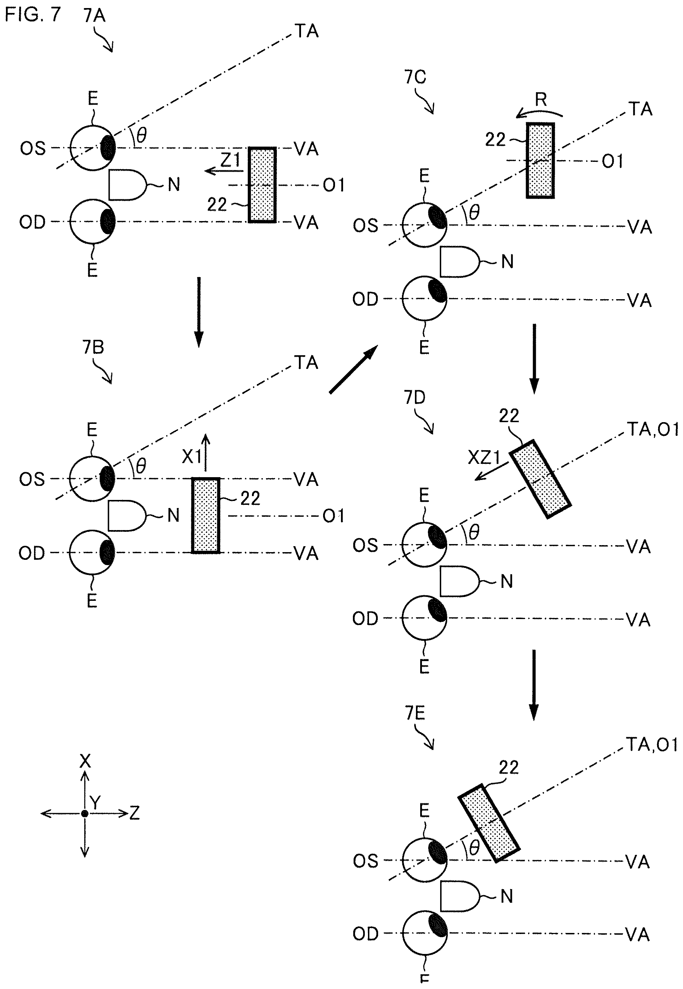

Figure 7 is an explanatory diagram for explaining an example 1-2 of the automatic alignment of the examination head 22 according to the first embodiment. The drive controlling unit 46 drives the XZ movement mechanism 16 to execute a first driving process of first moving the examination head 22 toward the front side (the subject eye E side) in the Z direction by a predetermined distance (see an arrow Z1), as indicated by reference character 7A in Figure 7. The drive controlling unit 46 then moving the examination head 22 to the axis TA of tilt in the outward direction X1, as indicated by reference character 7B in Figure 7. A distance of movement of the examination head 22 toward the front side in the Z direction is not particularly limited as long as a safe distance can be secured between the examination head 22 and the nose N. For example, the distance of movement may be determined based on a result of computing a Z-direction distance from the examination head 22 to the nose N based on photographed images obtained through stereoscopic photographing of the nose N by the stereo camera 34.

Moving the examination head 22 first toward the front side in the Z direction and then in the outward direction X1 to the tilt axis TA can reduce a moving distance of the examination head 22 along the direction X1, compared with the example 1-1 illustrated in Figure 6 described earlier.

As indicated by reference character 7C in Figure 7, the drive controlling unit 46 drives the swing rotation mechanism 20 after completion of the first driving process to execute the same second driving process as the example 1-1 (see reference character 6B in Figure 6) and make the optical axis O1 parallel to the axis TA of tilt. That the second driving process may be executed before the first driving process in the example 1-2 as well.

As indicated by reference character 7D in Figure 7, the drive controlling unit 46 drives the XZ movement mechanism 16 after completion of the second driving process to execute a third driving process in the same manner as the example 1-1 (see reference characters 6C and 6D in Figure 6), thereby moving the examination head 22 to the examination position along the axis TA of tilt when the examination head 22 is viewed from the one-direction side in the Y direction. As indicated by reference character 7E in Figure 7, the drive controlling unit 46 drives the XZ movement mechanism 16 and the Y movement mechanism 18 based on alignment detection performed by the alignment detection unit 44 halfway through the automatic alignment to continue the third driving process until the examination head 22 reaches the examination position.

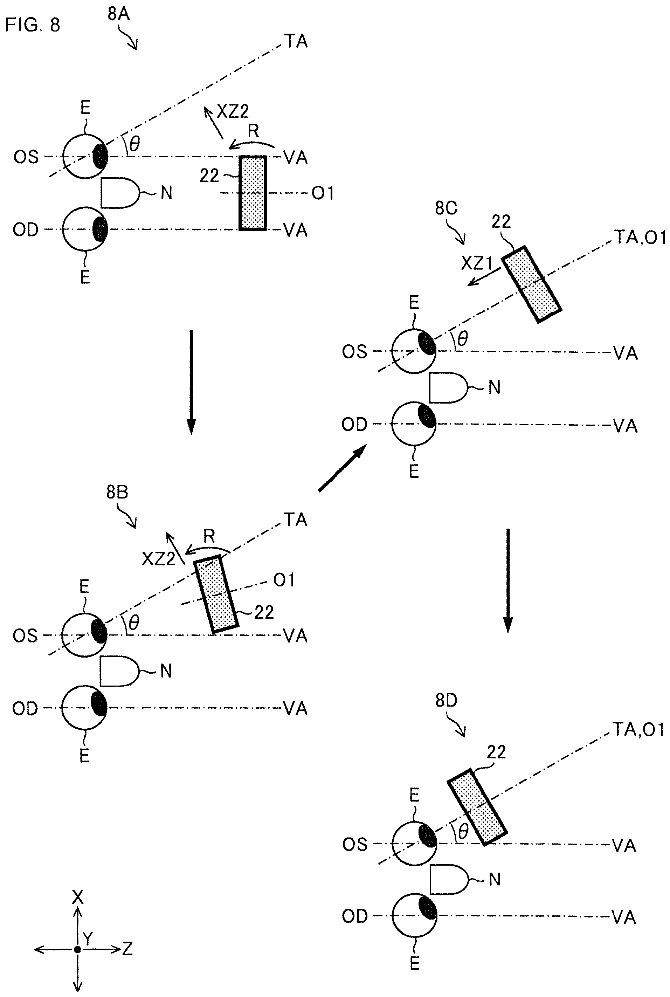

Figure 8 is an explanatory diagram for explaining an example 1-3 of the automatic alignment of the examination head 22 according to the first embodiment. As indicated by reference characters 8A and 8B in Figure 8, the drive controlling unit 46 drives the XZ movement mechanism 16, the Y movement mechanism 18, and the swing rotation mechanism 20 to execute a first driving process of simultaneously executing movement of the examination head 22 toward the front side in the Z direction and in the outward direction X1 and rotation of the examination head 22 by the tilting angle θ (see an arrow XZ2 and the arrow R). With this process, it is possible to move the examination head 22 to the axis TA of tilt in an oblique direction when the examination head 22 is viewed from the one-direction side in the Y direction and make the optical axis O1 parallel to the axis TA of tilt.

In the first driving process described in the example 1-3, the examination head 22 may be displaced to a position on the axis TA of tilt where the stereo camera 34 can photograph the anterior eye part of the subject eye E or to a position where an observation optical system (not illustrated) inside the examination head 22 can photograph the anterior eye part of the subject eye E via a shortest route.

As indicated by reference character 8C in Figure 8, the drive controlling unit 46 drives the XZ movement mechanism 16 and the Y movement mechanism 18 after completion of the first driving process to execute a second driving process. The second driving process in the example 1-3 is the same process as the third driving processes in the example 1-1 and the example 1-2, and moves the examination head 22 to the examination position along the axis TA of tilt when the examination head 22 is viewed from the one-direction side in the Y direction. As indicated by reference character 8D in Figure 8, the drive controlling unit 46 drives the XZ movement mechanism 16 and the Y movement mechanism 18 based on alignment detection performed by the alignment detection unit 44 halfway through the automatic alignment to continue the second driving process until the examination head 22 reaches the examination position.

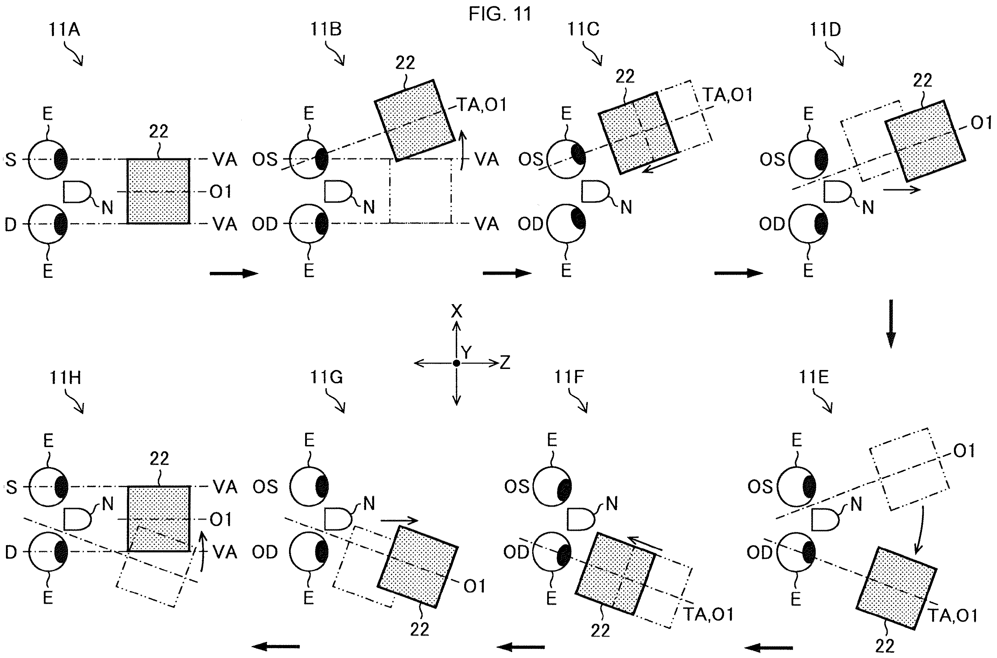

Referring back to Figure 4, when examination on the subject eye E by the examination head 22 (fundus imaging of the subject eye E by the fundus camera unit 24 or OCT imaging of the subject eye E by the OCT unit 26) is completed, the drive controlling unit 46 drives the XZ movement mechanism 16 to retract the examination head 22 toward a rear side in the Z direction (an examiner side) by a predetermined distance (see reference characters 11D and 11G in Figure 11 (to be described later)).

The vision fixation controlling unit 48 causes the vision fixation light emitting unit 36 to emit vision fixation light at least during a period from before the start of the automatic alignment of the examination head 22 to completion of examination on the subject eye E by the examination head 22. This makes it possible to guide and fix an eye direction of the subject to a direction of the vision fixation light during movement of the examination head 22 from the initial position to the examination position through the axis TA of tilt at the time of the automatic alignment. For this reason, when the examination head 22 is moved from the initial position to the axis TA of tilt, the subject eye E can be made to circumnutate so as to follow the movement (see Figures 6 to 8). As a result, the eye direction of the subject eye E can be kept fixed to the examination head 22.

The measurement controlling unit 50 controls fundus imaging of the subject eye E by the fundus camera unit 24 and photographing of a tomographic image of the subject eye E by the OCT unit 26. For example, the measurement controlling unit 50 drives a focus optical system (not illustrated) (see Patent Literature 1) housed in the examination head 22 after completion of the automatic alignment of the examination head 22 to execute an autofocusing process of focusing the examination head 22 on a part to be observed (e.g., the fundus) of the subject eye E. The measurement controlling unit 50 then executes fundus photographing of the subject eye E by the fundus camera unit 24 or OCT imaging of the subject eye E by the OCT unit 26.

The measurement controlling unit 50 acquires a fundus image of the subject eye E from the fundus camera unit 24 and outputs the fundus image to the saving controlling unit 52 when fundus imaging of the subject eye E by the fundus camera unit 24 is executed. The measurement controlling unit 50 generates a tomographic image of the subject eye E based on a signal such as a detection signal output from the OCT unit 26 by a publicly known method and outputs the tomographic image to the saving controlling unit 52 when OCT imaging of the subject eye E by the OCT unit 26 is executed.

The saving controlling unit 52 causes the display unit 37 to display a fundus image or a tomographic image of the subject eye E input from the measurement controlling unit 50. The saving controlling unit 52 saves the fundus image or the tomographic image of the subject eye E in the storage unit 39 when the examiner instructs an image saving using the manipulation unit 38.

### [Function of Ophthalmic Device According to First Embodiment]

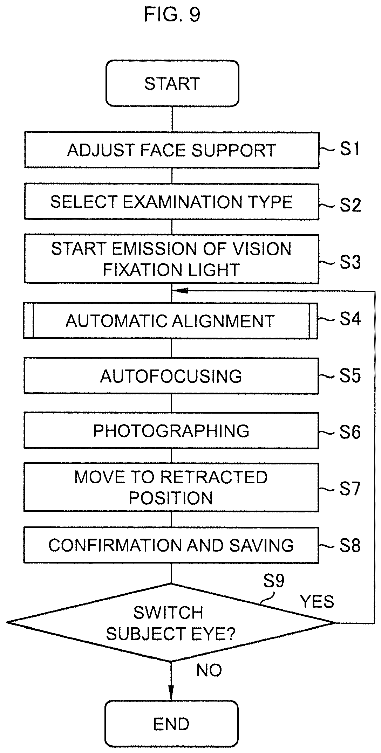

Figure 9 is a flowchart illustrating a flow of a process of examining the subject eye E by the ophthalmic device 10 according to the first embodiment with the above-described configuration. As illustrated in Figure 9, the examiner manipulates the manipulation unit 38 with the subject placing his/her chin against the chin rest 14a and his/her forehead against the forehead rest 14b to adjust height positions (positions in the Y direction) of the chin rest 14a and the forehead rest 14b to fit the subject (step S1).

The examiner then manipulates the manipulation unit 38 to select the type of examination on the subject eye E (fundus imaging by the fundus camera unit 24 or OCT imaging by the OCT unit 26) (step S2). The examiner also manipulates the manipulation unit 38 to select an automatic alignment mode as an alignment mode of the examination head 22. When the examination type of the subject eye E and the alignment mode are selected, the vision fixation controlling unit 48 causes the vision fixation light emitting unit 36 to emit vision fixation light (step S3). This allows guiding and fixation of the eye direction of the subject eye E.

When the examiner inputs an examination start instruction by using the manipulation unit 38, the automatic alignment of the examination head 22 with the subject eye E is executed (step S4).

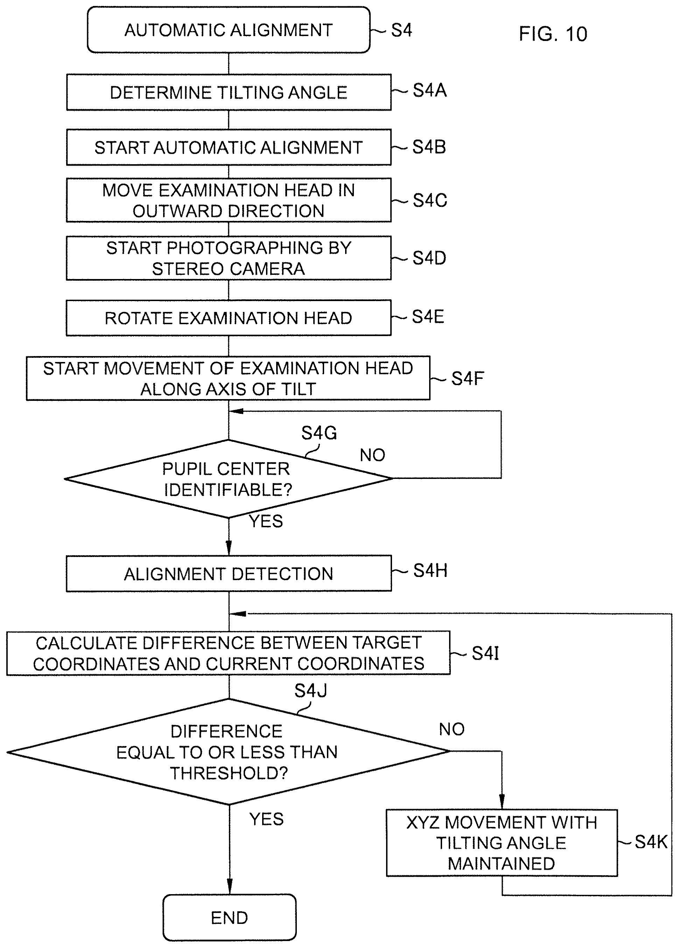

Figure 10 is a flowchart illustrating a flow of an automatic alignment process for the examination head 22 which pertains to a method for operating an ophthalmic device according to the presently disclosed subject matter. Figure 11 is an explanatory diagram for explaining displacement of the examination head 22 after the start of the automatic alignment. Here, the subject eye E is the left eye OS here and that a case where the automatic alignment in the example 1-1 illustrated in Figure 6 is executed will be described as an example.

As illustrated in Figures 10 and 11, after the tilting angle determining unit 43 determines, as the tilting angle θ, an angle selected in advance with the manipulation unit 38 by the examiner, the tilting angle determining unit 43 outputs information on the tilting angle θ to the drive controlling unit 46 (step S4A). For this reason, after the drive controlling unit 46 determines the axis TA of tilt based on the tilting angle θ, the drive controlling unit 46 starts the automatic alignment of the examination head 22 (step S4B).

The drive controlling unit 46 drives the XZ movement mechanism 16 to first execute a first driving process of moving the examination head 22 from the initial position to the axis TA of tilt in the outward direction X1 when the examination head 22 is viewed from the one-direction side in the Y direction (step S4C). The alignment detection unit 44 causes the cameras 34a of the stereo camera 34 to start photographing and continuously executes acquisition of photographed images from the cameras 34a and analysis of the photographed images (step S4D).

The drive controlling unit 46 then drives the swing rotation mechanism 20 to execute a second driving process of rotating the examination head 22 around the rotating shaft 20a by the tilting angle θ and making the optical axis O1 of the examination head 22 parallel to the axis TA of tilt (step S4E). With this process, the examination head 22 is displaced from the initial position indicated by reference character 11A in Figure 11 to the axis TA of tilt, as indicated by reference character 11B, and the optical axis O1 becomes parallel to the axis TA of tilt. Additionally, emission of vision fixation light from the vision fixation light emitting unit 36 allows the eye direction of the subject eye E to follow displacement of the examination head 22.

The drive controlling unit 46 drives the XZ movement mechanism 16 to start a third driving process of moving the examination head 22 to the examination position along the axis TA of tilt when the examination head 22 is viewed from the one-direction side in the Y direction (step S4F), as indicated by reference character 11C in Figure 11.

While the first driving process to the third driving process are executed, the alignment detection unit 44 waits for a chance for alignment detection (NO in step S4G) until the pupil center position of the subject eye E can be identified from photographed images acquired from the cameras 34a. Halfway through the automatic alignment, the cameras 34a photograph the anterior eye part of the subject eye E, and anterior eye part images of the subject eye E are input as photographed images from the cameras 34a to the alignment detection unit 44. This allows the alignment detection unit 44 to identify the pupil center position of the subject eye E based on the anterior eye part images input from the cameras 34a (YES in step S4G).

The alignment detection unit 44 then executes alignment detection that detects the relative position of the subject eye E to the examination head 22 by converting the pupil center position of the subject eye E into three-dimensional coordinates (step S4H). The alignment detection unit 44 outputs a detection result of the alignment detection to the drive controlling unit 46.

The drive controlling unit 46 drives the XZ movement mechanism 16 and the Y movement mechanism 18 based on the alignment detection result input from the alignment detection unit 44 to continue the third driving process until the examination head 22 reaches the examination position. Specifically, the drive controlling unit 46 calculates a difference between three-dimensional coordinates (target coordinates) of the examination position determined based on the alignment detection result and three-dimensional coordinates of the current examination head 22 (current coordinates) and continues the third driving process until the difference is equal to or less than a threshold (step S4I, NO in step S4J, and step S4K). In this manner, the examination head 22 is moved to the examination position while maintaining the tilting angle θ.

When the difference between the target coordinates and the current coordinates is equal to or less than the threshold, the drive controlling unit 46 stops driving the XZ movement mechanism 16 and the Y movement mechanism 18 to end the automatic alignment (YES in step S4J). The above-described displacement of the examination head 22 to the examination position along the axis TA of tilt at the time of the automatic alignment prevents the examination head 22 from coming close to the nose N.

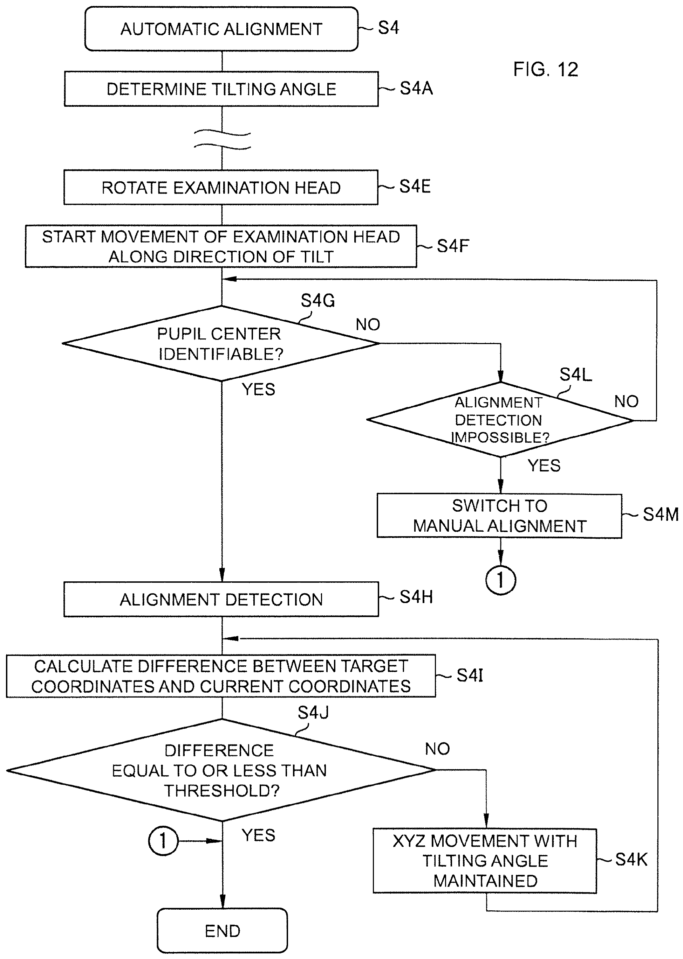

Figure 12 is a flowchart illustrating a flow of a modification of the automatic alignment of the examination head 22. Although alignment detection by the alignment detection unit 44 is executed halfway through the automatic alignment in Figure 10, the alignment detection unit 44 may fail to perform alignment detection even when the examination head 22 reaches a region which allows alignment detection.

For the above-described reason, as illustrated in Figure 12, the drive controlling unit 46 determines that alignment detection is impossible (NO in step S4G and YES in step S4L) in a case where the alignment detection unit 44 is incapable of identifying the pupil center position of the subject eye E during a period from the start of the automatic alignment or the start of the third driving process to movement of the examination head 22 for a fixed time determined in advance or over a fixed distance. In this case, the drive controlling unit 46 switches the alignment mode of the examination head 22 from the automatic alignment mode to a manual alignment mode and displays a message to that effect on the display unit 37 (step S4M). Based on the message, the examiner manipulates the manipulation unit 38 to execute manual alignment of the examination head 22. Since a switch to the manual alignment can be made halfway through the automatic alignment, the examination head 22 is prevented from coming close to the subject eye E in a state where alignment detection is impossible.

Referring back to Figures 9 and 11, when the automatic alignment is completed, the measurement controlling unit 50 drives the focus optical system (not illustrated) to execute autofocusing (step S5). After that, the measurement controlling unit 50 executes fundus imaging of the subject eye E by the fundus camera unit 24 or OCT imaging of the subject eye E by the OCT unit 26 (step S6). The measurement controlling unit 50 outputs a fundus image of the subject eye E acquired from the fundus camera unit 24 to the saving controlling unit 52 when fundus imaging is executed. The measurement controlling unit 50 generates a tomographic image of the subject eye E based on a signal such as a detection signal output from the OCT unit 26 and outputs the tomographic image to the saving controlling unit 52 when OCT imaging is executed.

When fundus imaging or OCT imaging of the subject eye E is completed, the drive controlling unit 46 drives the XZ movement mechanism 16 to retract the examination head 22 toward the rear side in the Z direction (step S7), as indicated by reference character 11D in Figure 11.

The saving controlling unit 52 causes the display unit 37 to display the fundus image or the tomographic image of the subject eye E input from the measurement controlling unit 50. This allows the examiner to confirm whether a desired fundus image or tomographic image is obtained. In a case where a desired fundus image or tomographic image is obtained, the examiner inputs an image saving manipulation to the manipulation unit 38. With this manipulation, the saving controlling unit 52 saves the fundus image or tomographic image of the subject eye E in the storage unit 39 (step S8).

In a case where the examiner proceeds to examine the right eye OD, the processes in step S4 to step S8 are repeatedly executed (YES in step S9). In this case, the examination head 22 is displaced to the axis TA of tilt corresponding to the right eye OD (see reference character 11E in Figure 11) and is further moved to an examination position for the right eye OD along the axis TA of tilt (see reference character 11F in Figure 11), under control by the drive controlling unit 46. When the examination on the right eye OD is completed, the examination head 22 is retracted toward the rear side in the Z direction (see reference character 11G in Figure 11) and is then displaced to the initial position (see reference character 11H in Figure 11), under the control by the drive controlling unit 46.

As described above, in the ophthalmic device 10 according to the first embodiment, the examination head 22 can be moved to the examination position for the subject eye E from an oblique direction along the axis TA of tilt at the time of the automatic alignment. For this reason, a sufficient distance can always be secured between the examination head 22 and the nose N as compared to a case where the examination head 22 is moved from a position in front of the subject eye E to an examination position on the front side in the Z direction (see reference character 5A in Figure 5). As a result, it is possible to examine the subject eye E by the examination head 22 without bringing the examination head 22 close to the nose N.

### [Second Embodiment]

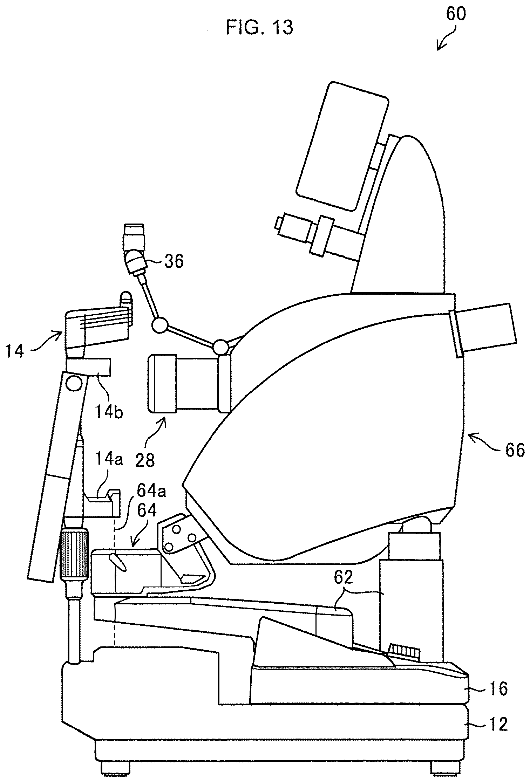

Figure 13 is a side view of an ophthalmic device 60 according to a second embodiment. While the examination head 22 is rotated (swung) around the objective lens 30 by the swing rotation mechanism 20 (the rotating shaft 20a) in the ophthalmic device 10 according to the above-described first embodiment, the ophthalmic device 60 according to the second embodiment includes an examination head 66 different in rotation center position from that in the first embodiment.

Components functionally or structurally identical to those in the ophthalmic device 10 according to the first embodiment are denoted by identical reference numerals, and a description thereof will be omitted.

As illustrated in Figure 13, the ophthalmic device 60 is a fundus camera and includes a base 12, a face support 14, an XZ movement mechanism 16, a vision fixation light emitting unit 36 (only an external fixation lamp of which is illustrated), a Y movement mechanism 62, a swing rotation mechanism 64, and the examination head 66. The ophthalmic device 60 also includes a stereo camera 34, a display unit 37, a manipulation unit 38, a storage unit 39, and a control device 40 (all not illustrated) described in the first embodiment.

The Y movement mechanism 62, together with the XZ movement mechanism 16, constitutes a movement mechanism according to the presently disclosed subject matter. The Y movement mechanism 62 has a shape extending toward a front side in a Z direction. The swing rotation mechanism 64 is provided at a distal end portion on the front side in the Z direction of the Y movement mechanism 62. The Y movement mechanism 62 integrally moves the swing rotation mechanism 64 and the examination head 66 in a Y direction. The XZ movement mechanism 16 and the Y movement mechanism 62 are capable of integrally moving the swing rotation mechanism 64 and the examination head 66 in an X direction and the Y and Z directions.

The swing rotation mechanism 64 corresponds to a rotation mechanism according to the presently disclosed subject matter and, together with the XZ movement mechanism 16 and the Y movement mechanism 62, constitutes a displacement mechanism according to the presently disclosed subject matter. The swing rotation mechanism 64 has a rotation axis 64a parallel to the Y direction and rotates the examination head 66 around the rotation axis 64a. The rotation axis 64a is provided in front of a lens-barrel 28 (an objective lens 30) of the examination head 66 in the Z direction. With this configuration, the rotation axis 64a and a subject eye E (a circumnutation center) can be made to coincide when viewed from a one-direction side in the Y direction by adjusting X and Z positions of the swing rotation mechanism 64 by the XZ movement mechanism 16. In this case, the swing rotation mechanism 64 rotates (swings) the examination head 66 around the circumnutation center of the subject eye E.

Although not illustrated, the swing rotation mechanism 64 can also rotate (tilt) the examination head 66 around a rotation axis perpendicular to the Y direction.

The examination head 66 is attached to the swing rotation mechanism 64. With this configuration, the examination head 66 is movable in the X, Y, and Z directions by the XZ movement mechanism 16 and the Y movement mechanism 62 and is rotatable in a direction around the rotation axis 64a by the swing rotation mechanism 64. The examination head 66 includes a fundus camera unit 24 and the lens-barrel 28 (including the stereo camera 34) described in the first embodiment.

The control device 40 according to the second embodiment is basically the same as the control device 40 according to the first embodiment except that a method for automatic alignment of the examination head 66 by a drive controlling unit 46 is different from that in the first embodiment.

The drive controlling unit 46 according to the second embodiment determines an axis TA of tilt based on a tilting angle θ determined by a tilting angle determining unit 43 and then drives the XZ movement mechanism 16, the Y movement mechanism 62, and the swing rotation mechanism 64 to execute the automatic alignment of the examination head 66, like the first embodiment.

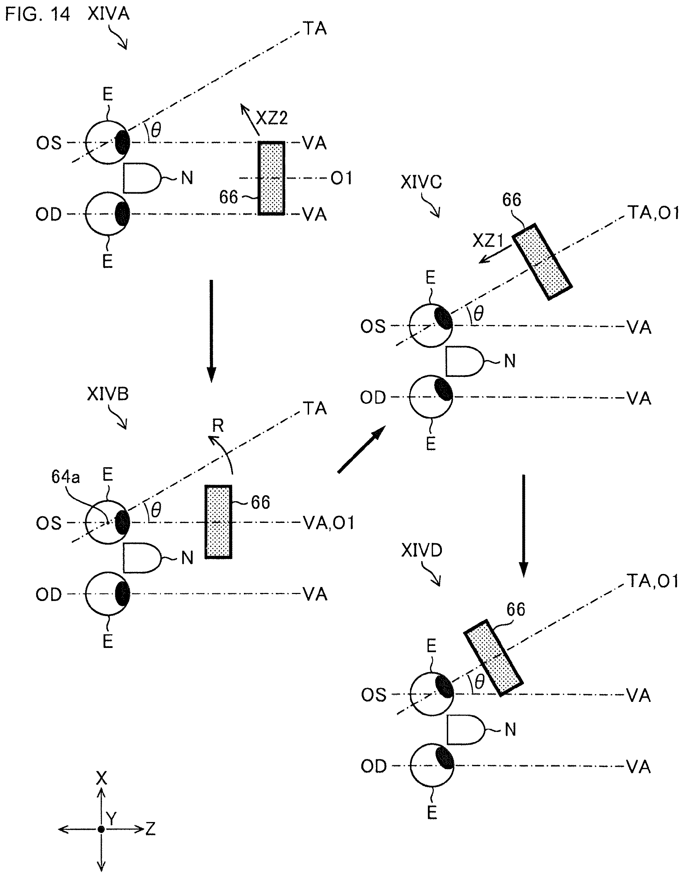

Figure 14 is an explanatory diagram for explaining an example 2-1 of the automatic alignment of the examination head 66 according to the second embodiment. As indicated by reference character XIVA in Figure 14, the examination head 66 is arranged at the same initial position as the first embodiment at first.

As indicated by reference characters XIVA and XIVB in Figure 14, the drive controlling unit 46 drives the XZ movement mechanism 16 to execute a first driving process of moving the examination head 66 (the swing rotation mechanism 64) from the initial position in the X and Z directions (see an arrow XZ2). Specifically, the examination head 66 is moved in the X and Z directions to a position where the rotation axis 64a coincides with the circumnutation center of the subject eye E when viewed from the one-direction side in the Y direction.

The drive controlling unit 46 drives the swing rotation mechanism 20 after completion of the first driving process to execute a second driving process of rotating the examination head 66 around the rotating shaft 20a (the circumnutation center of the subject eye E) by the tilting angle θ (see an arrow R). With this process, as indicated by reference character XIVC in Figure 14, the examination head 66 is moved to the axis TA of tilt, and an optical axis O1 becomes parallel to the axis TA of tilt.

The drive controlling unit 46 then drives the XZ movement mechanism 16 after completion of the second driving process to start a third driving process of moving the examination head 66 to an examination position along the axis TA of tilt when the examination head 66 is viewed from the one-direction side in the Y direction (see an arrow XZ1), like the first embodiment. In the third driving process according to the second embodiment, Z-axis movement of the examination head 66 by the XZ movement mechanism 16 is mainly executed, unlike the first embodiment. With this movement, the examination head 66 is moved toward the subject eye E while keeping the tilting angle θ constant.

As indicated by reference character XIVD in Figure 14, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

As described with reference to Figure 12, the drive controlling unit 46 switches an alignment mode of the examination head 66 to a manual alignment mode in a case where the alignment detection unit 44 is incapable of alignment detection during a period from the start of the automatic alignment or the start of the third driving process to movement of the examination head 66 for a fixed time determined in advance or over a fixed distance.

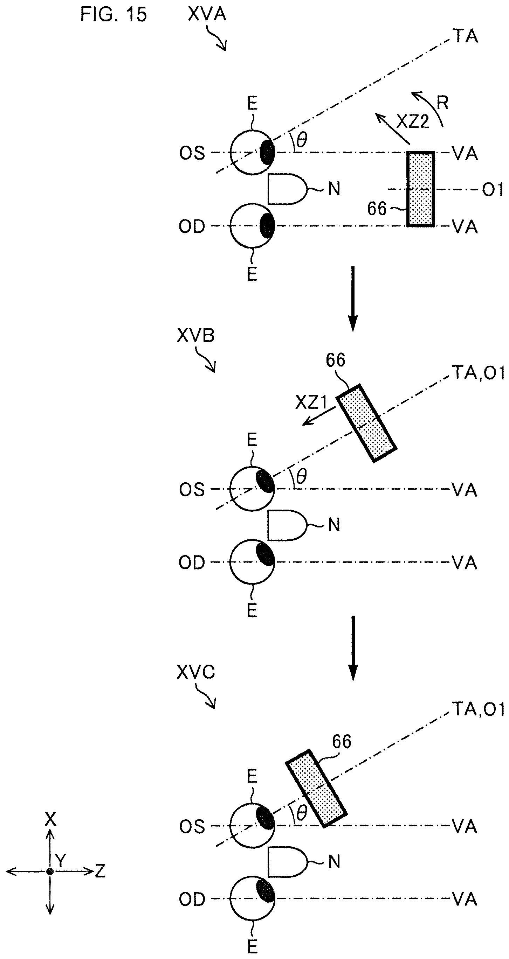

Figure 15 is an explanatory diagram for explaining an example 2-2 of the automatic alignment of the examination head 66 according to the second embodiment. As indicated by reference characters XVA and XVB in Figure 15, the drive controlling unit 46 simultaneously drives the XZ movement mechanism 16, the Y movement mechanism 62, and the swing rotation mechanism 64 to execute a first driving process of simultaneously executing movement of the examination head 66 in the X and Z directions and rotation of the examination head 66 by the tilting angle θ (see the arrow XZ2 and the arrow R). The first driving process in the example 2-2 is a process of simultaneously executing the first driving process and the second driving process in the example 2-1 described with reference to Figure 14. With this process, the examination head 66 is moved to the axis TA of tilt, and the optical axis O1 of the objective lens 30 becomes parallel to the axis TA of tilt.

In the first driving process, the examination head 66 may be displaced to a position on the axis TA of tilt where the stereo camera 34 can photograph an anterior eye part of the subject eye E or a position where an observation optical system (not illustrated) inside the examination head 22 can photograph the anterior eye part of the subject eye E via a shortest route.

The drive controlling unit 46 drives the XZ movement mechanism 16 and the Y movement mechanism 62 after completion of the first driving process to execute a second driving process which is the same as the third driving process in the example 2-1, thereby moving the examination head 66 to the examination position along the axis TA of tilt when the examination head 66 is viewed from the one-direction side in the Y direction (see the arrow XZ1). As indicated by reference character XVC in Figure 15, the drive controlling unit 46 drives the XZ movement mechanism 16 and the Y movement mechanism 62 based on alignment detection performed by the alignment detection unit 44 halfway through the automatic alignment to continue the second driving process until the examination head 66 reaches the examination position.

As described above, in the ophthalmic device 10 according to the second embodiment, the examination head 66 can be moved to the examination position for the subject eye E from an oblique direction along the axis TA of tilt at the time of the automatic alignment, like the first embodiment. The same effects as those of the first embodiment can be achieved.

### [Third Embodiment]

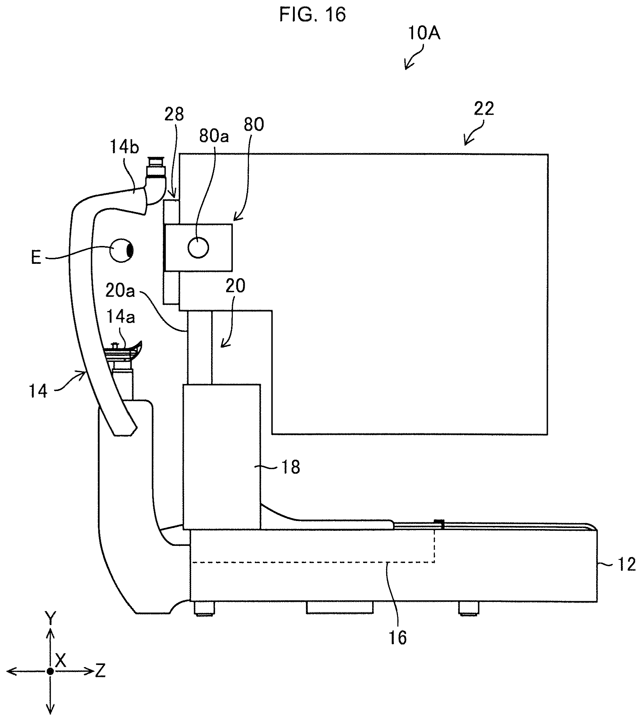

Figure 16 is a side view of an ophthalmic device 10A according to a third embodiment. The ophthalmic device 10 (see Figure 1) according to the above-described first embodiment includes the swing rotation mechanism 20 having the rotating shaft 20a parallel to the Y direction, and brings the examination head 22 close to the subject eye E along the axis TA of tilt obtained by tilting the reference axis VA in the X direction (the outward direction X1) around the subject eye E at the time of automatic alignment of the examination head 22. In contrast, the ophthalmic device 10A according to the third embodiment brings an examination head 22 close to a subject eye E along an axis TA of tilt obtained by tilting a reference axis VA in a direction other than an X direction around the subject eye E at the time of automatic alignment of the examination head 22.

As illustrated in Figure 16, the ophthalmic device 10A according to the third embodiment is basically the same in configuration as the ophthalmic device 10 according to the first embodiment except that the ophthalmic device 10A includes a tilt rotation mechanism 80 and executes the automatic alignment of the examination head 22 differently from the first embodiment. For this reason, components functionally or structurally identical to those in the ophthalmic device 10 according to the first embodiment are denoted by identical reference numerals, and a description thereof will be omitted.

The tilt rotation mechanism 80 corresponds to a rotation mechanism according to the presently disclosed subject matter and, together with an XZ movement mechanism 16, a Y movement mechanism 18, and a swing rotation mechanism 20, constitutes a displacement mechanism according to the presently disclosed subject matter. The tilt rotation mechanism 80 includes a rotating shaft 80a parallel to a Y direction and an electric drive mechanism which rotates the rotating shaft 80a, and rotates (tilts) the examination head 22 around the rotating shaft 80a.

A position of the rotating shaft 80a and a position of an objective lens 30 coincide (the term "coincide" as used herein is intended to include the meaning of "coincide substantially"; the same applies hereinafter) when the rotating shaft 80a is viewed from a one-direction side in an axial direction of the rotating shaft 80a. With this configuration, the examination head 22 is rotated (tilted) around the objective lens 30 by the tilt rotation mechanism 80.

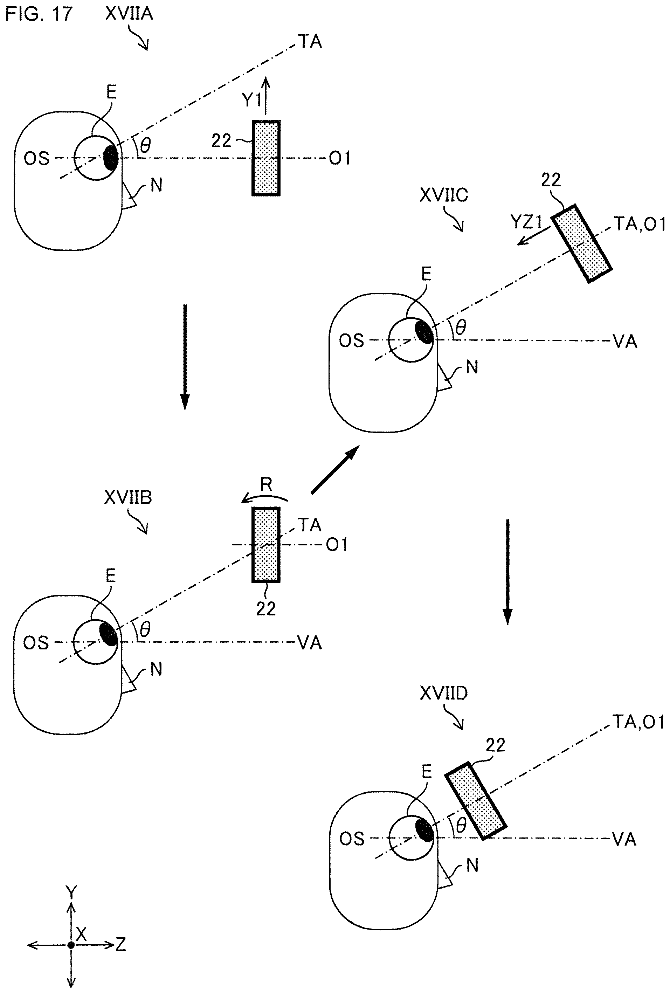

As described above, the examination head 22 according to the third embodiment is biaxially rotatable (swingable and tiltable) around the objective lens 30 by the swing rotation mechanism 20 and the tilt rotation mechanism 80. The examination head 22 according to the third embodiment is thus rotatable around an arbitrary rotating shaft (including ones other than a rotating shaft 20a and the rotating shaft 80a) perpendicular to a Z direction by driving at least one of the swing rotation mechanism 20 and the tilt rotation mechanism 80. For this reason, in the third embodiment, a direction (which is a direction perpendicular to the Z direction and away from a nose N) other than the outward direction X1 according to the first embodiment, such as an upward direction of the Y direction, is set as an outward direction Y1 (see Figure 17), and an axis obtained by tilting the reference axis VA in the outward direction Y1 around the subject eye E is set as the axis TA of tilt.

A control device 40 according to the third embodiment is basically the same as the control device 40 according to the first embodiment except that a direction of tilt of the axis TA of tilt is different from that in the first embodiment.

A tilting angle determining unit 43 according to the third embodiment determines a tilting angle θ in the outward direction Y1 (see Figure 17) of the axis TA of tilt with respect to the reference axis VA when viewed from a one-direction side in the X direction, i.e., the tilting angle θ of the axis TA of tilt with respect to the reference axis VA in a YZ plane. A specific method for determining the tilting angle θ is the same as the method for determining the tilting angle θ according to the first embodiment except that the direction of tilt of the axis TA of tilt is different and that a specific description thereof will be omitted.

A drive controlling unit 46 according to the third embodiment determines the axis TA of tilt based on the tilting angle θ determined by the tilting angle determining unit 43 and then drives the XZ movement mechanism 16, the Y movement mechanism 18, the swing rotation mechanism 20, and the tilt rotation mechanism 80 to execute the automatic alignment of the examination head 22.

Figure 17 is an explanatory diagram for explaining an example 3 of the automatic alignment of the examination head 22 according to the third embodiment. The example 3 is basically the same as the example 1-1 (see Figure 6) described in the first embodiment except that the direction of tilt of the axis TA of tilt is different.

As indicated by reference character XVIIA in Figure 17, the examination head 22 is arranged at the same initial position as the first embodiment at first. The drive controlling unit 46 according to the third embodiment drives the XZ movement mechanism 16 and the Y movement mechanism 18 to execute a first driving process of moving the examination head 22 from the initial position to the axis TA of tilt in the outward direction Y1 (the upward direction of the Y direction) when the examination head 22 is viewed from the one-direction side in the X direction.

As indicated by reference character XVIIB in Figure 17, the drive controlling unit 46 drives the tilt rotation mechanism 80 after completion of the first driving process to execute a second driving process of rotating the examination head 22 around the rotating shaft 80a by the tilting angle θ (see an arrow R). With this process, as indicated by reference character XVIIC in Figure 17, an optical axis O1 of the examination head 22 (the objective lens 30) becomes parallel to the axis TA of tilt. The second driving process may be executed before the first driving process.

The drive controlling unit 46 then drives the XZ movement mechanism 16 and the Y movement mechanism 18 after completion of the second driving process to start a third driving process of moving the examination head 22 to an examination position along the axis TA of tilt when the examination head 22 is viewed from the one-direction side in the X direction (see an arrow YZ1). With this process, the examination head 22 is moved toward the subject eye E while keeping the tilting angle θ constant (the term "constant" as used herein is intended to include the meaning of "substantially constant"; the same applies hereinafter).

As indicated by reference character XVIID in Figure 17, the drive controlling unit 46 drives the XZ movement mechanism 16 and the Y movement mechanism 18 based on alignment detection performed by an alignment detection unit 44 halfway through the automatic alignment to continue the third driving process until the examination head 22 reaches the examination position.

The drive controlling unit 46 switches an alignment mode of the examination head 22 to a manual alignment mode in a case where the alignment detection unit 44 is incapable of alignment detection during a period from the start of the automatic alignment or the start of the third driving process to movement of the examination head 22 for a fixed time determined in advance or over a fixed distance.

Like the example 1-2 (see Figure 7) of the automatic alignment of the examination head 22 according to the first embodiment, the first driving process may be started after the examination head 22 is first moved toward a front side in the Z direction (a subject eye E side) by a predetermined distance. Like the example 1-3 (see Figure 8) of the automatic alignment of the examination head 22 according to the first embodiment, a first driving process of simultaneously executing movement of the examination head 22 toward the front side in the Z direction and in the outward direction Y1 and rotation of the examination head 22 by the tilting angle θ may be executed.

As described above, in the ophthalmic device 10 according to the third embodiment as well, the examination head 22 can be moved to the examination position for the subject eye E from an oblique direction (obliquely upward direction) along the axis TA of tilt at the time of the automatic alignment. This prevents the examination head 22 from coming close to the nose N. As a result, the same effects as those of the first embodiment can be achieved.

Although the examination head 22 is brought close to the subject eye E along the axis TA of tilt obtained by tilting the reference axis VA in the outward direction Y1 (the upward direction of the Y direction) around the subject eye E at the time of the automatic alignment of the examination head 22 in the third embodiment, the direction of tilt of the axis TA of tilt is not particularly limited as long as the direction is a direction perpendicular to the Z direction and away from the nose N. Directions of the rotating shafts 20a and 80a may be appropriately changed in accordance with the direction of tilt.

In a case where the direction of tilt of the axis TA of tilt is fixed to the outward direction Y1 in the ophthalmic device 10A according to the third embodiment, the swing rotation mechanism 20 may be omitted.

### [Fourth Embodiment]

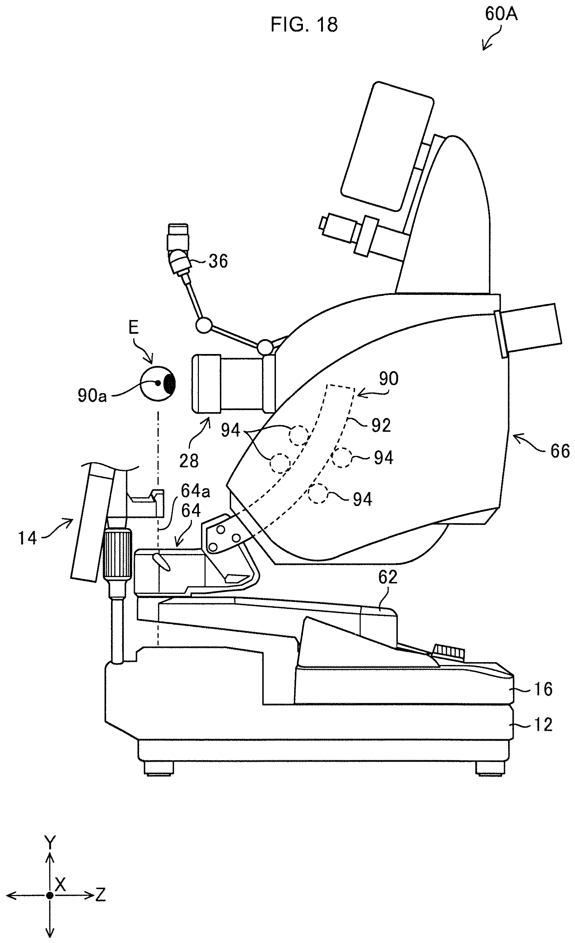

Figure 18 is a side view of an ophthalmic device 60A according to a fourth embodiment. The ophthalmic device 10 (see Figure 13) according to the above-described second embodiment includes the swing rotation mechanism 64 having the rotation axis 64a (corresponding to a first rotation axis according to the presently disclosed subject matter) parallel to the Y direction, and brings the examination head 66 close to the subject eye E along the axis TA of tilt obtained by tilting the reference axis VA in the X direction (the outward direction X1) around the subject eye E at the time of the automatic alignment of the examination head 66. In contrast, the ophthalmic device 60A according to the fourth embodiment brings an examination head 66 close to a subject eye E along an axis TA of tilt obtained by tilting a reference axis VA in a direction other than an X direction around the subject eye E at the time of automatic alignment of the examination head 66, like the ophthalmic device 10A according to the above-described third embodiment.

As illustrated in Figure 18, the ophthalmic device 60A according to the fourth embodiment is basically the same in configuration as the ophthalmic device 60 according to the second embodiment except that the ophthalmic device 60A includes a tilt rotation mechanism 90 and executes the automatic alignment of the examination head 66 differently from the second embodiment. For this reason, components functionally or structurally identical to those in the ophthalmic device 60 according to the second embodiment are denoted by identical reference numerals, and a description thereof will be omitted.

The tilt rotation mechanism 90 corresponds to a rotation mechanism according to the presently disclosed subject matter and, together with an XZ movement mechanism 16, a Y movement mechanism 62, and a swing rotation mechanism 64, constitutes a displacement mechanism according to the presently disclosed subject matter. The tilt rotation mechanism 90 rotates (tilts) the examination head 66 around an imaginary rotation axis 90a (corresponding to a second rotation axis according to the presently disclosed subject matter) perpendicular to a Y direction.

The tilt rotation mechanism 90 includes, for example, a curved arm 92, a plurality of guide wheel portions 93, and a head moving mechanism (not illustrated). The curved arm 92 is fixed to the swing rotation mechanism 64 and has the shape of an arc of a circle centered at the rotation axis 90a.

The plurality of guide wheel portions 93 are held inside the examination head 66 so as to be rotatable around center axes parallel to the rotation axis 90a. The guide wheel portions 93 are arranged such that the curved arm 92 is sandwiched therebetween in the Y direction. With this configuration, the examination head 66 is movable along the curved arm 92.

The head moving mechanism (not illustrated) of the tilt rotation mechanism 90 is provided inside the examination head 66 and moves the examination head 66 along the curved arm 92. A configuration of the head moving mechanism is a publicly known technique (e.g., Japanese Patent Application Laid-Open No. 2022-112637) and that a specific description thereof will be omitted. The movement of the examination head 66 along the curved arm 92 by the head moving mechanism allows rotation (tilting) of the examination head 66 around the rotation axis 90a. Additionally, alignment of the rotation axis 90a with the subject eye E allows rotation (tilting) of the examination head 66 around the subject eye E.

As described above, the examination head 66 according to the fourth embodiment is biaxially rotatable (swingable and tiltable) by the swing rotation mechanism 64 and the tilt rotation mechanism 90, like the examination head 22 according to the third embodiment. The examination head 66 according to the fourth embodiment is thus rotatable around an arbitrary rotation axis (including ones other than a rotation axis 64a and the rotation axis 90a) perpendicular to a Z direction by driving at least one of the swing rotation mechanism 64 and the tilt rotation mechanism 90. For this reason, in the fourth embodiment, a direction (which is a direction perpendicular to the Z direction and away from a nose N) other than the outward direction X1 according to the second embodiment, such as an upward direction of the Y direction, is set as an outward direction Y1, and an axis obtained by tilting the reference axis VA in the outward direction Y1 around the subject eye E is set as the axis TA of tilt, like the third embodiment.

A control device 40 according to the fourth embodiment is basically the same as the control device 40 according to the second embodiment except that a direction of tilt of the axis TA of tilt is different from that in the second embodiment.

A tilting angle determining unit 43 according to the fourth embodiment determines a tilting angle θ in the outward direction Y1 of the axis TA of tilt with respect to the reference axis VA, like the tilting angle determining unit 43 according to the third embodiment.

A drive controlling unit 46 according to the fourth embodiment determines the axis TA of tilt based on the tilting angle θ determined by the tilting angle determining unit 43 and then drives the XZ movement mechanism 16, the Y movement mechanism 62, the swing rotation mechanism 64, and the tilt rotation mechanism 90 to execute the automatic alignment of the examination head 66.

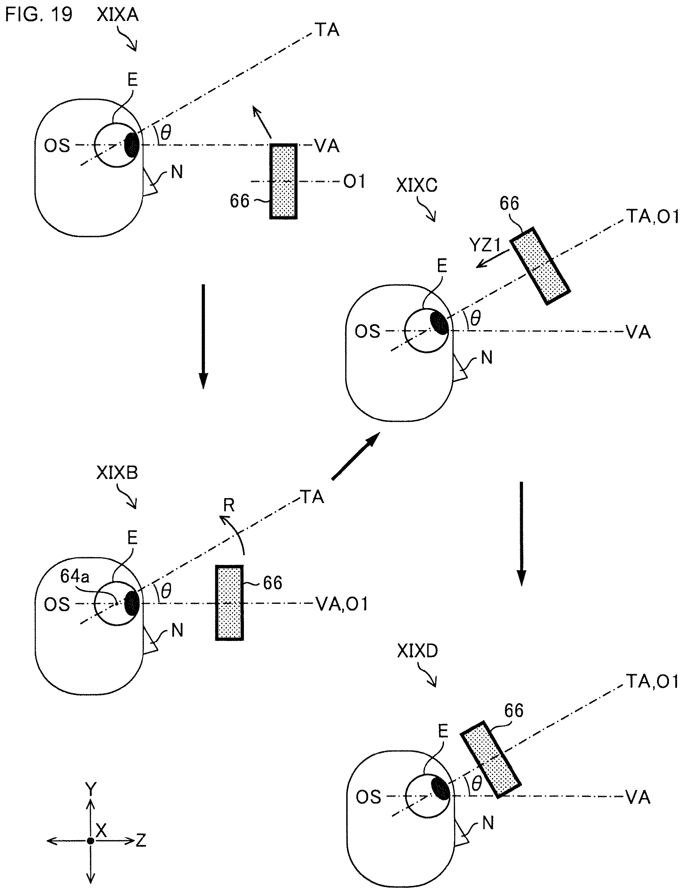

Figure 19 is an explanatory diagram for explaining an example 4 of the automatic alignment of the examination head 66 according to the fourth embodiment. The example 4 is basically the same as the example 2-1 (see Figure 14) described in the second embodiment except that the direction of tilt of the axis TA of tilt is different. As indicated by reference character XIXA in Figure 19, the examination head 66 is arranged at the same initial position as the first embodiment at first.

As indicated by reference characters XIXA and XIXB in Figure 19, the drive controlling unit 46 drives the XZ movement mechanism 16 and the Y movement mechanism 62 to execute a first driving process of moving, in the X, Y, and Z directions, the examination head 66 to a position where the rotation axis 64a and the rotation axis 90a coincide with a circumnutation center of the subject eye E.

The drive controlling unit 46 drives the tilt rotation mechanism 90 after completion of the first driving process to execute a second driving process of rotating the examination head 66 in the outward direction Y1 around the rotation axis 90a (the circumnutation center of the subject eye E) by the tilting angle θ (see an arrow R). With this process, as indicated by reference character XIXC in Figure 19, the examination head 66 is moved to the axis TA of tilt, and an optical axis O1 becomes parallel to the axis TA of tilt.

The drive controlling unit 46 then drives the XZ movement mechanism 16 and the Y movement mechanism 62 after completion of the second driving process to start a third driving process of moving the examination head 66 to an examination position along the axis TA of tilt when the examination head 66 is viewed from a one-direction side in the X direction (see an arrow YZ1). With this process, the examination head 66 is moved toward the subject eye E while keeping the tilting angle θ constant (the term "constant" as used herein is intended to include the meaning of "substantially constant"; the same applies hereinafter).

As indicated by reference character XIXD in Figure 19, the drive controlling unit 46 drives the XZ movement mechanism 16 and the Y movement mechanism 62 based on alignment detection performed by an alignment detection unit 44 halfway through the automatic alignment to continue the third driving process until the examination head 66 reaches the examination position.

The drive controlling unit 46 switches an alignment mode of the examination head 66 to a manual alignment mode in a case where the alignment detection unit 44 is incapable of alignment detection during a period from the start of the automatic alignment or the start of the third driving process to movement of the examination head 66 for a fixed time determined in advance or over a fixed distance.

The first driving process and the second driving process may be simultaneously executed, like the example 2-2 (see Figure 15) of the automatic alignment of the examination head 66 according to the second embodiment.

As described above, in the ophthalmic device 10 according to the fourth embodiment as well, the examination head 66 can be moved to the examination position for the subject eye E from an oblique direction (obliquely upward direction) along the axis TA of tilt at the time of the automatic alignment. This prevents the examination head 66 from coming close to the nose N. As a result, the same effects as those of the second embodiment can be achieved.

Although the examination head 66 is brought close to the subject eye E along the axis TA of tilt obtained by tilting the reference axis VA in the outward direction Y1 (the upward direction of the Y direction) around the subject eye E at the time of the automatic alignment of the examination head 66 in the fourth embodiment, the direction of tilt of the axis TA of tilt around the subject eye E is not particularly limited as long as the direction is a direction perpendicular to the Z direction and away from the nose N. Directions of the rotation axes 64a and 90a may be appropriately changed in accordance with the direction of tilt.

In a case where the direction of tilt of the axis TA of tilt is fixed to the outward direction Y1 in the ophthalmic device 10A according to the fourth embodiment, the swing rotation mechanism 64 may be omitted.

### [Others]

Although alignment detection is executed using the stereo camera 34 in each of the above-described embodiments, the alignment detection may be executed using an observation optical system (not illustrated) (corresponding to a photographing unit according to the presently disclosed subject matter) housed in the examination head 22 or 66. In this case, the stereo camera 34 may be omitted.

Although the stereo camera 34 is provided at the lens-barrel distal end face 28a in each embodiment, the stereo camera 34 may be provided at a position other than ones at the lens-barrel distal end face 28a in the examination head 22 or 66.

Although the examination head 22 or 66 is moved from an oblique direction to the examination position for the subject eye E along the axis TA of tilt
at the time of automatic alignment in each embodiment, the examination head 22 or 66 may be moved from an oblique direction to the examination position for the subject eye E along the axis TA of tilt at the time of manual alignment.

Although a case where a displacement mechanism which displaces the examination head 22 or 66 with respect to the subject eye E includes the XZ movement mechanism 16, the Y movement mechanism 18 or 62, and the swing rotation mechanism 20 or 64 and a case where the displacement mechanism includes the tilt rotation mechanism 80 or 90 in addition to the listed components have been described as examples in the embodiments, a configuration and the type of the displacement mechanism are not particularly limited. For example, a robot arm (multijoint arm) may be used as a displacement mechanism according to the presently disclosed subject matter.

Although a multifunction machine which is a combination of a fundus camera and an optical coherence tomograph and a fundus camera alone have been described as examples of the ophthalmic device 10 in the embodiments, the presently disclosed subject matter is not limited to this. The presently disclosed subject matter can also be applied to various ophthalmic devices (including devices which perform various procedures on the subject eye E, such as a laser surgery device) which are used for examination (ocular characteristics measurement, photographing, and observation) on the subject eye E and execute alignment of various examination heads with the subject eye E, such as an optical coherence tomograph alone and an SLO device.

### {Reference Signs List}

10 ophthalmic device
12 base
14 face support
14a chin rest
14b forehead rest
16 XZ movement mechanism
18 Y movement mechanism
20 swing rotation mechanism
20a rotating shaft
22 examination head
24 fundus camera unit
26 OCT unit
28 lens-barrel
28a lens-barrel distal end face
30 objective lens
32 illumination light source
34 stereo camera
34a camera
36 vision fixation light emitting unit
37 display unit
38 manipulation unit
39 storage unit
40 control device
43 tilting angle determining unit
44 alignment detection unit
46 drive controlling unit
48 vision fixation controlling unit
50 measurement controlling unit
52 saving controlling unit
60 ophthalmic device
62 Y movement mechanism
64 swing rotation mechanism
64a rotation axis
66 examination head
80, 90 tilt rotation mechanism
80a, 90a rotating shaft, rotation axis
100 objective lens
102 objective lens
104 examination head
E subject eye
N nose
O1 optical axis
OD right eye
OS left eye
TA axis of tilt
VA reference axis
X1 outward direction
θ tilting angle

## Claims

1. An ophthalmic device comprising:
an examination head configured to examine a subject eye; and
a displacement mechanism configured to displace the examination head with respect to the subject eye,
wherein the displacement mechanism is configured to displace the examination head to an examination position for the subject eye along an axis of tilt,
wherein the axis of tilt is an axis obtained by tilting, around the center of the subject eye, a reference axis that extends along the eye direction of the subject eye in parallel with a front-back direction serving as an operating distance direction of the examination head, outward away from a subject's nose.

2. The ophthalmic device according to claim 1, wherein the displacement mechanism displaces the examination head to the examination position while keeping a tilting angle of the axis of tilt with respect to the reference axis constant.

3. The ophthalmic device according to claim 2, wherein
the displacement mechanism includes
a movement mechanism configured to move the examination head in the front-back direction, a left-right direction, and an up-down direction with respect to the subject eye, and
a rotation mechanism configured to rotate the examination head around a rotation axis determined in advance.

4. The ophthalmic device according to claim 3, wherein
the rotation axis is parallel to the up-down direction, and
the outward direction is parallel to the left-right direction.

5. The ophthalmic device according to claim 3, wherein
the rotation axis is perpendicular to the up-down direction, and
the outward direction is an upward direction of the up-down direction.

6. The ophthalmic device according to claim 3, wherein
the examination head includes an objective lens,
the rotation mechanism rotates the examination head around the objective lens, and
the movement mechanism integrally moves the examination head and the rotation axis in the front-back direction, the left-right direction, and the up-down direction.

7. The ophthalmic device according to claim 6, wherein
the rotation axis is parallel to the up-down direction, and the outward direction is parallel to the left-right direction,
an optical axis of the objective lens is parallel to the front-back direction and is located between respective reference axes constituting for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of driving the movement mechanism to move the examination head from the initial position to the axis of tilt in the outward direction,
a second driving process of driving the rotation mechanism to rotate the examination head around the rotation axis and make the optical axis of the objective lens parallel to the axis of tilt, and
a third driving process of driving the movement mechanism after completion of the first driving process and the second driving process to move the examination head to the examination position along the axis of tilt.

8. The ophthalmic device according to claim 6, wherein
the rotation axis is parallel to the up-down direction, and the outward direction is parallel to the left-right direction,
an optical axis of the objective lens is parallel to the front-back direction and is located between respective reference axes constituting for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of driving the movement mechanism to move the examination head from the initial position toward a front side in the front-back direction which faces the subject eye and then move the examination head to the axis of tilt in the outward direction,
a second driving process of driving the rotation mechanism to rotate the examination head around the rotation axis and make the optical axis of the objective lens parallel to the axis of tilt, and
a third driving process of driving the movement mechanism after completion of the first driving process and the second driving process to move the examination head to the examination position along the axis of tilt.

9. The ophthalmic device according to claim 6, wherein
the rotation axis is parallel to the up-down direction, and the outward direction is parallel to the left-right direction,
an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of driving the movement mechanism and the rotation mechanism to simultaneously execute movement of the examination head toward a front side in the front-back direction which faces the subject eye, movement of the examination head in the outward direction, and rotation of the examination head around the rotation axis, and move the examination head to the axis of tilt and make the optical axis of the objective lens parallel to the axis of tilt, and
a second driving process of driving the movement mechanism after completion of the first driving process to move the examination head to the examination position along the axis of tilt.

10. The ophthalmic device according to claim 6, wherein
the rotation axis is perpendicular to the up-down direction, and the outward direction is an upward direction of the up-down direction,
an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of driving the movement mechanism and the rotation mechanism to move the examination head to the axis of tilt and make the optical axis of the objective lens parallel to the axis of tilt, and
a second driving process of driving the movement mechanism after completion of the first driving process to move the examination head to the examination position along the axis of tilt.

11. The ophthalmic device according to claim 3, wherein
the examination head includes an objective lens,
the rotation axis is located on a front side in the front-back direction which is closer to the subject eye than the objective lens is, and
the movement mechanism integrally moves the examination head and the rotation axis in the front-back direction, the left-right direction, and the up-down direction.

12. The ophthalmic device according to claim 11, wherein
the rotation axis is parallel to the up-down direction, and the outward direction is parallel to the left-right direction,
an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of driving the movement mechanism to move the examination head to a position where the rotation axis coincides with the subject eye when the examination head is viewed from the one-direction side,
a second driving process of driving the rotation mechanism after completion of the first driving process to move the examination head to the axis of tilt and make the optical axis of the objective lens parallel to the axis of tilt by rotating the examination head around the rotation axis, and
a third driving process of driving the movement mechanism after completion of the second driving process to move the examination head to the examination position along the axis of tilt.

13. The ophthalmic device according to claim 11, wherein
the rotation axis is parallel to the up-down direction, and the outward direction is parallel to the left-right direction,
an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of simultaneously executing a process of driving the movement mechanism to move the examination head to a position where the rotation axis coincides with the subject eye when the examination head is viewed from the one-direction side and a process of driving the rotation mechanism to rotate the examination head around the rotation axis, and moving the examination head to the axis of tilt and making the optical axis of the objective lens parallel to the axis of tilt, and
a second driving process of driving the movement mechanism after completion of the first driving process to move the examination head to the examination position along the axis of tilt.

14. The ophthalmic device according to claim 11, wherein the rotation mechanism is capable of rotating the examination head around a first rotation axis and a second rotation axis constituting the rotation axis, the first rotation axis being parallel to the up-down direction, the second rotation axis being perpendicular to the up-down direction.

15. The ophthalmic device according to claim 14, wherein
an optical axis of the objective lens is parallel to the front-back direction and is located between respective reference axes constituting the reference axis for left and right subject eyes constituting the subject eye at an initial position for the examination head when the examination head is viewed from a one-direction side in the up-down direction,
the ophthalmic device comprises a drive controlling unit configured to control driving of the displacement mechanism, and
the drive controlling unit executes
a first driving process of executing a process of driving the movement mechanism to move the examination head to a position where the first rotation axis and the second rotation axis coincide with the subject eye and a process of driving the rotation mechanism to rotate the examination head around at least one of the first rotation axis and the second rotation axis, and moving the examination head to the axis of tilt and making the optical axis of the objective lens parallel to the axis of tilt, and
a second driving process of driving the movement mechanism after completion of the first driving process to move the examination head to the examination position along the axis of tilt.

16. The ophthalmic device according to any one of claims 1 to 15, comprising
a vision fixation light emitting unit configured to emit vision fixation light.

17. The ophthalmic device according to any one of claims 1 to 15, comprising:
a photographing unit provided at the examination head and configured to photograph an anterior eye part of the subject eye halfway through displacement of the examination head by the displacement mechanism;
an alignment detection unit configured to detect a relative position of the subject eye to the examination head based on an anterior eye part image of the subject eye photographed by the photographing unit; and
a drive controlling unit configured to control driving of the displacement mechanism, wherein
the drive controlling unit drives the displacement mechanism based on a detection result from the alignment detection unit to move the examination head to the examination position along the axis of tilt.

18. The ophthalmic device according to claim 17, comprising
a manipulation unit, wherein
the drive controlling unit is switchable to a manual control mode of driving the displacement mechanism in accordance with an input manipulation to the manipulation unit to displace the examination head, and
the drive controlling unit switches to the manual control mode in a case where detection of the relative position by the alignment detection unit is not executed while the examination head is moved for a fixed time determined in advance or over a fixed distance by the displacement mechanism.

19. The ophthalmic device according to claim 17, wherein the photographing unit comprises a plurality of cameras capable of photographing the subject eye from a plurality of directions different from each other.

20. A method for operating an ophthalmic device, the ophthalmic device including
an examination head configured to examine a subject eye, and
a displacement mechanism including a rotation mechanism configured to displace the examination head with respect to the subject eye,
the method including driving the displacement mechanism to displace the examination head to an examination position for the subject eye along an axis of tilt, wherein the axis of tilt is an axis obtained by tilting, around the center of the subject eye, a reference axis that extends along the eye direction of the subject eye in parallel with a front-back direction serving as an operating distance direction of the examination head, outward away from a subject's nose.
